(19) **Europäisches Patentamt**
**European Patent Office**
**Office européen des brevets**

(11) **EP 2 502 966 A1**

(12) **EUROPÄISCHE PATENTANMELDUNG**

(43) Veröffentlichungstag:
26.09.2012 Patentblatt 2012/39

(51) Int Cl.:
*C09C 1/00* (2006.01)     *A61K 8/02* (2006.01)
*A61K 8/19* (2006.01)     *A61Q 1/02* (2006.01)

(21) Anmeldenummer: **12161116.4**

(22) Anmeldetag: **23.03.2012**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **25.03.2011 DE 102011001579**

(71) Anmelder: **Eckart GmbH**
**91235 Hartenstein (DE)**

(72) Erfinder:
• **Grüner, Michael**
**91275 Auerbach (DE)**

• **Schneider, Thomas**
**91207 Lauf a. d. Pegnitz (DE)**
• **Kaupp, Günter**
**91284 Neuhaus (DE)**
• **Rummer, Christian**
**90459 Nürnberg (DE)**
• **Schumacher, Dirk**
**91257 Pegnitz (DE)**

(74) Vertreter: **Walcher, Armin**
**Louis, Pöhlau, Lohrentz**
**Postfach 3055**
**90014 Nürnberg (DE)**

(54) **Kosmetische Formulierungen umfassend hochglänzende nichtmetallische silberfarbene Pigmente**

(57) Die vorliegende Erfindung betrifft eine kosmetische Formulierung umfassend ein kosmetisch akzeptables Medium und wenigstens ein silberfarbenes Pigment, wobei das silberfarbene Pigment ein nichtmetallisches plättchenförmiges Substrat und wenigstens eine ilmenithaltige Beschichtung umfasst.

Abbildung 1:

EP 2 502 966 A1

**Beschreibung**

Die vorliegende Erfindung betrifft eine kosmetische Formulierung umfassend ein kosmetisch akzeptables Medium und wenigstens ein silberfarbenes Pigment, wobei das silberfarbene Pigment ein nichtmetallisches plättchenförmiges Substrat und wenigstens eine ilmenithaltige Beschichtung umfasst.

Der Einsatz silberfarbener Effektpigmente in kosmetischen Formulierungen unterliegt hinsichtlich der Größe und Art des eingesetzten Pigments strengen regulatorischen Auflagen. Beispielsweise dürfen gemäß FDA Code of Federal Regulation Title 21, Volume 1, 73.1645 in den USA Aluminiumeffektpigmente, die ein Sieb mit einer 200 mesh Siebmaschenweite nicht vollständig passieren, nicht im Bereich Farbkosmetik eingesetzt werden. Des Weiteren ist es gemäß dieser Verordnung nicht gestattet Aluminiumeffektpigmente im Bereich der Lippe und des Mundes in kosmetischen Formulierungen einzusetzen. Somit sind beispielsweise globale Vermarktungskonzepte für Lippenstifte, die Aluminiumeffektpigmente enthalten, nicht möglich. Aus marktstrategischer Sicht ist eine solche Einschränkung unerwünscht.

Ist der metallische Effekt und der optische Eindruck eines Aluminiumeffektpigments in einer kosmetischen Formulierung erwünscht, so kann kein einfacher Ersatz des Aluminiumeffektpigments durch ein silberfarbenes Perlglanzpigment erfolgen. Hinsichtlich ihrer optischen Eigenschaften unterscheiden sich Metalleffektpigmente und Perlglanzpigmente grundlegend voneinander. Metalleffektpigmente weisen einen harten metallischen Spiegelglanz auf, wogegen Perlglanzpigmente einen weichen, scheinbar aus der Tiefe kommenden samtigen Glanz zeigen. Handelsübliche silberfarbene Perlglanzpigmente besitzen in der Regel nicht den für Aluminiumeffektpigmente charakteristischen neutralen Silberfarbton. Auch die Deckfähigkeit von opaken Metalleffektpigmenten kann von üblicherweise transparenten Perlglanzpigmenten nicht erzielt werden. Es besteht also ein Bedarf an Pigmenten für kosmetische Formulierungen, welche hinsichtlich der optischen Eigenschaften wie beispielsweise metallisch erscheinendem Silberfarbton, Deckung, metallischem Glanz oder Hell-/ Dunkelflop Metalleffektpigmenten nahe kommen, dennoch kein Metall, wie z.B. Aluminium, aufweisen. Des Weiteren sollen neben den bereits genannten Kriterien schwermetallfreie und lichtstabilere Alternativen zu silberfarbenen Pigmenten bestehend aus oder enthaltend Bismutoxychlorid zur Verfügung gestellt werden.

Der vorliegenden Erfindung lag die Aufgabe zugrunde, eine kosmetische Formulierung bereitzustellen, welche wasserstabile hochglänzende silberfarbene Pigmente, umfasst. Die silberfarbenen Pigmente sollen hinsichtlich ihres optischen Eindrucks die für Metalleffektpigmente charakteristischen Eigenschaften aufweisen. Die silberfarbenen Pigmente sollen sich in ihrem Aussehen nicht oder nur unwesentlich von handelsüblichen Aluminiumeffektpigmenten unterscheiden. Gleichzeitig sollen sich die silberfarbenen Pigmente durch eine hohe Stabilität, insbesondere gegenüber Säuren, Basen, acidischen oder alkalischen kosmetischen Inhaltsstoffen, wässrigen oder protisch/ aprotisch polaren Medien, sowie durch ihre Temperaturbeständigkeit auszeichnen. Des Weiteren sollen die in der kosmetischen Formulierung einsetzbaren silberfarbenen Pigmente rieselfähig sein und nicht zur Staubexplosion neigen.

Die der Erfindung zugrundeliegende Aufgabe wurde durch Bereitstellung einer kosmetischen Formulierung gelöst, welche ein kosmetisch akzeptables Medium und wenigstens ein silberfarbenes Pigment umfasst, wobei das silberfarbene Pigment ein nichtmetallisches plättchenförmiges Substrat und wenigstens eine ilmenithaltige Beschichtung umfasst.

Bevorzugte Weiterbildungen der erfindungsgemäßen kosmetischen Formulierung sind in den Unteransprüchen angegeben.

Gemäß einer bevorzugten Variante der Erfindung umfasst die kosmetische Formulierung ein silberfarbenes Pigment mit folgendem Aufbau:

> (a) nichtmetallisches plättchenförmiges Substrat,
> (b) Titanoxidschicht,
> (c) Ilmenitschicht,
> wobei das Pigment erhältlich ist durch

>> (i) Aufbringen einer nicht kalzinierten Titanoxid-/ Titanhydroxid/Titanoxidhydratschicht auf einem nichtmetallischen, plättchenförmigen, synthetischen Substrat,
>> (ii) Aufbringen einer Eisenoxid-/ Eisenhydroxid-/ Eisenoxidhydratschicht auf die nicht kalzinierte Titanoxid-/ Titanhydroxid-/ Titanoxidhydratschicht,
>> (iii) Kalzinieren des in Schritt (ii) erhaltenen Produktes unter reduzierenden Bedingungen.

Nach Schritt (iii) wird das silberfarbene Pigment erhalten.

Weiterhin wird die Aufgabe der Erfindung gelöst durch die Verwendung des silberfarbenen Pigments in einer kosmetischen Formulierung. Bevorzugte Weiterbildungen sind in den Ansprüchen 11 bis 15 angegeben.

Der Begriff kosmetisch akzeptables Medium umfasst beispielsweise Nagellackbasissysteme, Pulvermischungen, W/O-, O/W- oder W/Si-Emulsionen, Lotionen, Creme, Wachspräparationen, gesättigte und ungesättigte Öle, Tinten, Gele, Flüssigseife, Kernseife oder Shampoos. Hierbei kann es sich Vor- oder Halbfertigprodukte, wie beispielsweise Tönpasten, handeln oder die vorstehend genannten kosmetisch akzeptablen Medien können aus den entsprechenden kosmetischen Rohstoffen, nach dem Fachmann bekannten Methoden, hergestellt werden. Es kann sich aber auch um bereits pigmentierte und/ oder mit Füll- und/oder Hilfsstoffen versehene Endformulierungen handeln, in die nachträglich die silberfarbenen Pigmente eingearbeitet werden.

Mit Titanoxid-/ Titanhydroxid/ Titanoxidhydratschicht bzw. Eisenoxid-/Eisenhydroxid-/ Eisenoxidhydratschicht ist gemeint, dass eine Titanoxidschicht und/oder Titanhydroxidschicht und/ oder Titanoxidhydratschicht bzw. eine Eisenoxidschicht und/ oder Eisenhydroxidschicht und/ oder Eisenoxidhydratschicht vorliegen kann.

Unter Ilmenit wird im Sinne dieser Erfindung eine Verbindung der stöchiometrischen Zusammensetzung $FeTiO_3$ verstanden. Ilmenit kann auch als Eisentitanat bezeichnet werden.

Der Gehalt an Eisenverbindungen, berechnet als elementares Eisen, in dem silberfarbenen Pigment liegt bei weniger als 5,0 Gew.-%, bevorzugt in einem Bereich von 1 Gew.-% bis 4,3 Gew.-%, besonders bevorzugt in einem Bereich von 1,4 Gew.-% bis 2,9 Gew.-% und ganz besonders bevorzugt in einem Bereich von 1,5 Gew.-% bis 2,3 Gew.-%, jeweils bezogen auf das Gesamtgewicht des Pigments.

Unter Gehalt an Eisenverbindungen, nachfolgend auch als Eisengehalt bezeichnet, wird im Sinne dieser Erfindung der komplette Gehalt an Eisenverbindungen unterschiedlicher Oxidationszahlen im Pigment verstanden, wobei die Gehalte der Gesamtheit an nachweisbaren Eisenverbindungen auf elementares Eisen umgerechnet werden. Dies gilt sowohl für den Gehalt an Eisenverbindungen in den nichtmetallischen plättchenförmigen synthetischen Substraten als auch für den Gehalt an Eisenverbindungen in der Beschichtung.

Die Begriffe Schicht oder Beschichtung werden im Sinne dieser Erfindung austauschbar verwendet, sofern nicht anders angegeben.

Die Empfindung einer Farbe als matt, blass oder kräftig hängt maßgeblich von ihrer Farbsättigung, dem sogenannten Chroma oder der Buntheit ab. Dabei wird das Chroma durch die enthaltene Menge an Grau bestimmt. Je höher der Graugehalt, desto geringer ist die Farbsättigung.

Betrachtet man einen Punkt F im CIELab-Farbsystem, so ist dieser über die drei Koordinaten L* (Helligkeit), a* (rot-grün-Achse) und b* (gelb-blau-Achse) definiert. Die Farbkoordinaten a* und b* können auch über Polarkoordinaten C* (Chroma) und h* (Farbwinkel, Farbort) ausgedrückt werden, wobei die Definition wie folgt gegeben ist:

$$C^* = \sqrt{a^{*2} + b^{*2}}$$

$$h^* = \frac{180}{\pi} \cdot \arctan\left(\frac{b^*}{a^*}\right)$$

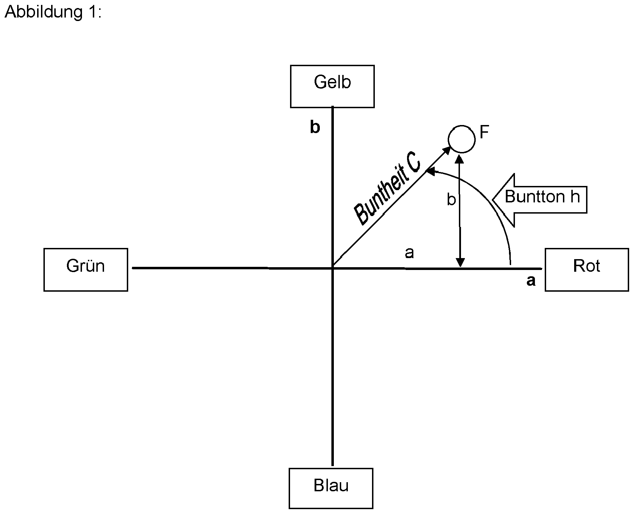

Das Chroma entspricht also der Länge des Vektors, der vom Ursprung des Koordinatensystems auf den zu definierenden Punkt F zeigt. Je geringer der C*-Wert ausfällt, desto näher liegt der Punkt F am unbunten Bereich des Farbkoordinatensystems. Das Chroma ist also der Abstand von der L*- oder Grau-Achse, die senkrecht auf der a*,b*-Ebene steht (Abbildung 1).

Die silberfarbenen Pigmente zeichnen sich durch geringe Chromawerte aus. Das Chroma liegt bei einer Messgeometrie von 110°, relativ zum Ausfallwinkel des bei 45° eingestrahlten Lichts, bei $C^*_{110} \leq 2,4$, bevorzugt in einem Bereich von $C^*_{110} = 0$ bis 2,3, besonders bevorzugt in einem Bereich von $C^*_{110} = 0,1$ bis 2,1 und ganz besonders bevorzugt in einem Bereich von $C^*_{110} = 0,2$ bis 1,9. Das Chroma liegt bei einer Messgeometrie von 75°, relativ zum Ausfallwinkel des bei 45° eingestrahlten Lichts, bei $C^*_{75} \leq 2,4$, bevorzugt in einem Bereich von $C^*_{75} = 0$ bis 2,3, besonders bevorzugt in einem Bereich von $C^*_{75} = 0,1$ bis 2,1 und ganz besonders bevorzugt in einem Bereich von $C^*_{75} = 0,2$ bis 1,9. Die Chromawerte werden mit dem Gerät Byk-mac, Fa. Byk-Gardner, anhand von Lackapplikationen auf Blechen gemessen.

Die Lackapplikationen auf Blechen wurden, wie nachfolgend in Abschnitt IVa beschrieben, hergestellt.

Weiterhin zeichnen sich die silberfarbenen Pigmente durch niedrige, nahe am Koordinatenursprung liegende, a*- und b*-Werte im CIELab-Farbsystem aus. Bevorzugte a*-Werte, gemessen anhand von Lackapplikationen auf Blechen mit einem Byk-mac, Fa. Byk-Gardner, über die, relativ zum Ausfallwinkel des bei 45° eingestrahlten Lichts, Messgeometrien von 15°, 25°, 45°, 75°, 110° liegen in einem Bereich von maximal +/- 2, bevorzugte b*-Werte bei diesen Messgeometrien liegen in einem Bereich von maximal +/- 4.

Werden die silberfarbenen Pigmente anhand von Pulverschüttungen vermessen, so zeichnen sich diese auch in nicht orientiertem Zustand durch niedrige a*- und b*-Werte und folglich auch durch niedrige Chromawerte aus.

Die nichtmetallischen plättchenförmigen synthetischen Substrate der silberfarbenen Pigmente sind vorzugsweise im Wesentlichen transparent, bevorzugt transparent, d.h. für sichtbares Licht sind sie zumindest teilweise durchlässig, bevorzugt durchlässig.

Die nichtmetallischen plättchenförmigen synthetischen Substrate können aus der Gruppe, bestehend aus synthetischen Glimmerplättchen, Glasplättchen, $SiO_2$-Plättchen, $Al_2O_3$-Plättchen, synthetischen Böhmitplättchen, Polymerplättchen, synthetischen plättchenförmigen Substraten, die eine anorganisch-organische Mischschicht umfassen, und deren Gemische, ausgewählt werden. Bevorzugt werden die nichtmetallischen plättchenförmigen synthetischen Substrate aus der Gruppe bestehend aus synthetischen Glimmerplättchen, Glasplättchen, $Al_2O_3$-Plättchen und deren Gemische, ausgewählt. Besonders bevorzugt werden die nichtmetallischen plättchenförmigen synthetischen Substrate aus der Gruppe bestehend aus synthetischen Glimmerplättchen, Glasplättchen und deren Gemische, ausgewählt. Insbesondere bevorzugt sind synthetische Glimmerplättchen als Substrat.

Im Unterschied zu nichtmetallischen plättchenförmigen synthetischen Substraten besitzen plättchenförmige natürliche Substrate den Nachteil, dass letztere Verunreinigungen durch eingelagerte Fremdionen enthalten können. Diese Verunreinigungen können den Farbton verändern und/ oder die Helligkeit L* herabsetzen. Typische Verunreinigungen von beispielsweise natürlichem Glimmer stellen u.a. Nickel, Chrom, Kupfer, Eisen, Mangan, Blei, Cadmium, Arsen und/ oder Antimon und/ oder deren Verbindungen dar, welche beispielsweise dem natürlichen Glimmer eine Färbung geben können.

Der Gehalt der vorgenannten Fremdionen, mit Ausnahme von Eisen, berechnet als elementares Metall, liegt in dem nichtmetallischen plättchenförmigen synthetischen Substrat vorzugsweise jeweils bei weniger als 15 ppm, weiter bevorzugt bei weniger als 10 ppm, jeweils bezogen auf das Gesamtgewicht des Substrates.

Insbesondere sollte der Eisengehalt, berechnet als elementares Eisen, der nichtmetallischen plättchenförmigen synthetischen Substrate möglichst niedrig sein und vorzugsweise bei weniger als 0,20 Gew.-%, bevorzugt in einem Bereich von 0,01 Gew.-% bis 0,20 Gew.-%, weiter bevorzugt in einem Bereich von 0,03 Gew.-% bis 0,19 Gew.-% und besonders bevorzugt in einem Bereich von 0,04 Gew.-% bis 0,18 Gew.-%, jeweils bezogen auf das Gesamtgewicht des Substrats, liegen.

Bevorzugt wird der Eisengehalt der nichtmetallischen plättchenförmigen synthetischen Substrate über Röntgenfluoreszenzanalyse (RFA) bestimmt. Hierzu werden die nichtmetallischen plättchenförmigen synthetischen Substrate mit Lithiumtetraborat versetzt, in oxidierender Atmosphäre geschmolzen und als homogene Glastablette vermessen. Als Messgerät diente das Gerät Advantix ARL, Fa. Thermo Scientific.

Neben der Farbneutralität des nichtmetallischen plättchenförmigen synthetischen Substrats ist auch dessen Helligkeit mitverantwortlich für den optischen Eindruck darauf basierender Pigmente. Die Helligkeit L* der nichtmetallischen plättchenförmigen synthetischen Substrate, bestimmt durch diffuse Farbmessung der jeweiligen Pulverschüttungen mit einem Farbmessgerät CR 310 der Firma Konica Minolta, liegt bevorzugt bei ≥ 90, besonders bevorzugt bei ≥ 92 und ganz besonders bevorzugt bei ≥ 95.

Ein weiterer Unterschied zwischen nichtmetallischen plättchenförmigen natürlichen und synthetischen Substraten ist, dass die Oberfläche plättchenförmiger natürlicher Substrate herstellungsbedingt nicht ideal glatt ist, sondern Unregelmäßigkeiten, wie z.B. Stufen, aufweisen kann. Nichtmetallische plättchenförmige synthetische Substrate weisen in der Regel glatte Oberflächen sowie eine einheitliche Dicke innerhalb eines einzelnen Substratteilchens sowie vorzugsweise über die Gesamtheit aller Substratteilchen auf. Somit bietet die Oberfläche nur wenig Streuzentren für einfallendes bzw. reflektiertes Licht und ermöglicht so nach Beschichtung dieser plättchenförmigen Substrate höher glänzende Pigmente als mit beispielsweise plättchenförmigem natürlichem Glimmer als Substrat.

Des Weiteren sind Verunreinigungen durch Schwermetalle, insbesondere in kosmetischen Formulierungen, im Interesse des Verbrauchers unerwünscht. Insbesondere erhöhte Bleigehalte in kosmetischen Formulierungen sind unerwünscht. Farbadditive werden bzgl. ihres Bleigehalts von der FDA überwacht und dürfen einen Grenzwert von 20 μg/ g nicht überschreiten. Weitere kosmetische Inhaltsstoffe unterliegen im Hinblick auf den Bleigehalt der Verantwortung der Hersteller (Nancy M. Hepp, William R. Mindak, John Cheng, J. Cosmet. Sci., 60, 405 - 414 (July/ August 2009)).

Bei einer Ausführungsform liegt der Bleigehalt der als Substrat einsetzbaren synthetischen Glimmerplättchen bei vorzugsweise weniger als 5 ppm, bevorzugt in einem Bereich von 0,05 ppm bis 3 ppm und besonders bevorzugt in einem Bereich von 0,03 ppm bis 2 ppm. Äußerst bevorzugt enthalten die synthetischen Glimmerplättchen kein Blei bzw. keine Bleiverbindungen.

Bei einer weiteren Ausführungsform weisen die auf synthetischen Glimmerplättchen basierenden, silberfarbenen Pigmente einen Gesamtbleigehalt von vorzugsweise weniger als 10 ppm, bevorzugt aus einem Bereich von 0,0 ppm bis weniger als 9 ppm, weiter bevorzugt aus einem Bereich von 0,0 ppm bis weniger als 8 ppm, noch weiter bevorzugt aus einem Bereich von 0,1 ppm bis weniger als 7 ppm und besonders bevorzugt aus einem Bereich von 0,1 ppm bis weniger als 6,5 ppm, auf.

Die Bestimmung des Bleigehalts der synthetischen Glimmerplättchen sowie der hierauf basierenden silberfarbenen Pigmente erfolgt hierbei über Feststoff-Graphitrohr-Atomabsorptionsspektrometrie. Als Gerät wird vorzugsweise ein ZEENIT 650 mit Feststoffprobengeber SSA 600, Fa. Analytik Jena, eingesetzt.

Bei einer weiteren Ausführungsform können die nichtmetallischen plättchenförmigen synthetischen Substrate einen Brechungsindex aus einem Bereich von 1,55 bis 1,70, bevorzugt aus einem Bereich von 1,58 bis 1,68 und besonders bevorzugt aus einem Bereich von 1,59 bis 1,65 aufweisen.

Besteht das nichtmetallische plättchenförmige synthetische Substrat aus Glasplättchen, so werden im Rahmen dieser Erfindung bevorzugt jene verwendet, die nach den in EP 0 289 240 A1, WO 2004/056716 A1 und der WO 2005/063637 A1 beschriebenen Verfahren hergestellt werden. Die als Substrat verwendbaren Glasplättchen können beispielsweise eine Zusammensetzung entsprechend der Lehre der EP 1 980 594 B1 aufweisen.

Besteht das nichtmetallische plättchenförmige synthetische Substrat aus synthetischem Glimmer kann dieser verschiedene chemische Zusammensetzungen aufweisen und sich unter anderem in seinen optischen Eigenschaften unterscheiden. Unterschiede im plättchenförmigen Substrat können auch im darauf basierenden Pigment wahrgenommen werden. Die Wahl des geeigneten synthetischen plättchenförmigen Glimmers als zu beschichtendes Substrat ist daher von großer Bedeutung für das optische Erscheinungsbild der resultierenden Pigmente.

Bei synthetischen Glimmerplättchen als Substrat handelt es sich im Rahmen dieser Erfindung vorzugsweise um Fluorphlogopit der allgemeinen Formel $X_1Y_{2-3}n(Z_4O_{10})F_2$, wobei X aus der Gruppe bestehend aus $K^+$, $Na^+$, $Li^+$ und/ oder $Ca^{2+}$, Y aus der Gruppe bestehend aus $Mg^{2+}$ und/ oder $Zn^{2+}$ und Z aus der Gruppe bestehend aus $Si^{4+}$ und/ oder $Al^{3+}$ ausgewählt werden kann und n für ½ oder 1 steht. Besonders bevorzugt wird Fluorphlogopit der Formel $KMg_3AlSi_3O_{10}F_2$, $KMg_2 1/2(Si_4O_{10})F_2$ oder $NaMg_2 1/2(Si_4O_{10})F_2$ als nichtmetallisches plättchenförmiges Substrat eingesetzt. Ganz besonders bevorzugt ist hierbei Fluorphlogopit der Formel $KMg_3AlSi_3O_{10}F_2$.

Plättchenförmiger Fluorphlogopit ist ein hochtemperaturbeständiges und chemikalienstabiles Substrat, das für die Zwecke der vorliegenden Erfindung sehr gut geeignet ist.

Die Herstellung des synthetischen Glimmers kann dabei gezielt gesteuert werden, so dass die resultierenden synthetischen Glimmerplättchen möglichst wenige Fehlstellen aufweisen.

Die als nichtmetallisches plättchenförmiges synthetisches Substrat bevorzugt verwendeten synthetischen Glimmerplättchen umfassen gemäß Röntgenfluoreszenzanalyse vorzugsweise die in Tabelle 1 genannten Bestandteile in den aufgeführten Bereichen.

Tabelle 1: Bevorzugte Zusammensetzungen von plättchenförmigem synthetischem Glimmer gemäß Röntgenfluoreszenzanalyse (RFA)

| Zusammensetzung synthetischer Glimmerplättchen, Angaben in Gew.-%, jeweils bezogen auf das Gesamtgewicht der synthetischen Glimmerplättchen | |
|---|---|
| $SiO_2$ | 38 bis 46 |
| $Al_2O_3$ | 10 bis 14 |
| $K_2O$ | 9 bis 13 |
| $Fe_2O_3$ | 0,01 bis 0,25 |
| MgO | 26 bis 34 |
| MnO | 0 bis 0,05 |
| $Na_2O$ | 0 bis 13 |

Auch bei geringfügigen Abweichungen von den in Tabelle 1 beispielhaft angegebenen Werten können die in der erfindungsgemäßen kosmetischen Formulierung einzusetzenden silberfarbenen Pigmente erhalten werden. Es versteht sich von selbst, dass hierbei der Anteil an färbenden Komponenten nicht signifikant von den in Tabelle 1 angegebenen Werten abweichen sollte bzw. keine weiteren oder nur unwesentliche Spuren an färbenden Komponenten im Substrat zugegen sein dürfen.

Bevorzugte Magnesiumoxidwerte der silberfarbenen Pigmente liegen gemäß Röntgenfluoreszenzanalyse in einem Bereich von 10 bis 30 Gew.-%, besonders bevorzugt in einem Bereich von 13 bis 27 Gew.-%, ganz besonders bevorzugt in einem Bereich von 17 bis 23 Gew.-%, jeweils bezogen auf das Gesamtgewicht der Pigmente.

Auf die nichtmetallischen plättchenförmigen synthetischen Substrate wird wenigstens eine hochbrechende Schicht mit einem Brechungsindex von n > 2,0, bevorzugt von n > 2,2, aufgebracht. Die wenigstens eine hochbrechende Schicht weist eine Metalloxidschicht und/ oder eine Metallhydroxidschicht und/ oder eine Metalloxidhydratschicht auf oder besteht daraus.

Zur Ausbildung einer Ilmenitschicht muss das nichtmetallische plättchenförmige synthetische Substrat sowohl wenigstens eine Titanoxid- als auch wenigstens eine angrenzende Eisenoxidschicht und/ oder wenigstens eine Titanhydroxid- und wenigstens eine angrenzende Eisenhydroxidschicht und/ oder wenigstens eine Titanoxidhydrat- und wenigstens eine angrenzende Eisenoxidhydratschicht umfassen. Unter reduzierenden Bedingungen, bevorzugt in Gegenwart von Formiergas ($N_2$/ $H_2$), und bei Temperaturen von wenigstens 500°C findet an der Grenzfläche zwischen Titanoxid- und Eisenoxidschicht bzw. Titanoxidhydrat- und Eisenoxidhydratschicht bzw. Titanhydroxid- und Eisenhydroxidschicht die Reaktion zu Ilmenit statt. Im Grenzbereich findet eine partielle Durchdringung beider Schichten unter Bildung von Ilmenit statt. In den resultierenden silberfarbenen Pigmenten wird entsprechend ein Gradient von einer ausschließlich aus Titanoxid zu einer ausschließlich aus Ilmenit bestehenden Schicht gefunden. In der nach anschließender Kalzination Titanoxid umfassenden Schicht können zusätzlich geringe Mengen an unter den Reduktionsbedingungen entstehenden Titansuboxidspezies vorhanden sein, wobei deren Anteil so gering ist, dass das Aussehen der silberfarbenen Pigmente hiervon nicht beeinflusst wird.

Gemäß einer bevorzugten Ausführungsform der Erfindung nimmt der Anteil an Titanoxid in der Beschichtung von der dem Substrat zugewandten Seite zur dem Substrat abgewandten Seite der Titanoxidschicht ab. Somit weist auch die Ilmenitschicht einen in Richtung Substrat abnehmenden Konzentrationsgradienten auf.

Um die silberfarbenen Pigmente zu erhalten, kann das zur Ilmenitbildung benötigte Titandioxid in der Anatas- oder Rutilform vorliegen. Bei einer bevorzugten Ausführungsform liegt das Titandioxid in der Rutilform vor. Die Rutilform kann erhalten werden, indem beispielsweise vor Aufbringung der Titandioxidschicht eine Schicht aus Zinndioxid auf das zu beschichtende plättchenförmige transparente Substrat aufgebracht wird. Auf dieser Schicht aus Zinndioxid kristallisiert Titandioxid in der Rutilmodifikation. Das Zinndioxid kann dabei als separate Schicht vorliegen, wobei die Schichtdicke wenige Nanometer, beispielsweise weniger als 10 nm, weiter bevorzugt weniger als 5 nm, noch weiter bevorzugt weniger als 3 nm, betragen kann.

Bei einer besonders bevorzugten Ausführungsform findet die Reaktion zu Ilmenit an der Grenzfläche der Titanoxidhydrat-/ Titanhydroxidschicht und Eisenoxidhydrat-/Eisenhydroxidschicht statt, d.h. ein mit Titanoxidhydrat und/ oder mit Titanhydroxid beschichtetes Pigment wird ohne vorherige Kalzination und ohne vorherige optionale Isolation mit Eisenoxidhydrat und/ oder Eisenhydroxid belegt und anschließend unter reduzierenden Bedingungen bei erhöhter Temperatur behandelt oder kalziniert.

Die in der erfindungsgemäßen kosmetischen Formulierung einzusetzenden silberfarbenen Pigmente zeichnen sich durch einen neutralen oder reinen Silberfarbton ohne Farbstich, beispielsweise ohne eine schwach blaue, grünliche, rötliche oder goldene Färbung aus, welcher optisch auf ein Perlglanzpigment schließen lassen könnte. Neutrale oder reine Silberfarbtöne sind charakteristisch für Metalleffektpigmente, wie beispielsweise Aluminiumeffektpigmente. Den silberfarbenen Pigmenten fehlt mithin das für Perlglanzpigmente charakteristische Auftreten von Interferenz- und Komplementärfarbe, welches insbesondere auf weißem Untergrund, in Abhängigkeit vom Betrachtungswinkel auftritt. Auch fehlt den silberfarbenen Pigmenten der für Perlglanzpigmente typische Tiefenglanz.

Verläuft die Reaktion zu Ilmenit unvollständig, d.h. ist nach der Reduktion noch Eisen(III)oxid vorhanden, so besitzen die resultierenden Pigmente eine bräunliche Färbung. Diese Abweichung von einem neutralen Silberfarbton kann mit bloßem Auge erkannt werden. Bei einer bevorzugten Ausführungsform weisen die silberfarbenen Pigmente einen Eisen(III)oxidgehalt von weniger als 0,5 Gew.-%, weiter bevorzugt aus einem Bereich von 0,0 Gew.-% bis 0,4 Gew.-%, noch weiter bevorzugt von weniger als 0,3 Gew.-%, besonders bevorzugt aus einem Bereich von 0,1 Gew.-% bis 0,3 Gew.-%, jeweils bezogen auf das Gesamtgewicht des Pigments, auf.

Aufgrund der Ilmenitschicht gehen typische Eigenschaften von Perlglanzpigmenten wie Tiefenglanz und Transparenz verloren. Vielmehr weisen die silberfarbenen Pigmente charakteristische Merkmale von Metalleffektpigmenten, wie die hervorragende Deckfähigkeit, auf.

Werden die silberfarbenen Pigmente hinsichtlich ihrer optischen Eigenschaften mit silberfarbenen Perlglanzpigmenten ohne Ilmenitschicht verglichen, so lässt sich feststellen, dass die silberfarbenen Pigmente bereits bei einem sehr geringen Ilmenitgehalt den optischen Eindruck eines Aluminiumeffektpigments erwecken. Die bei Perlglanzpigmenten ohne Ilmenitschicht vorhandene Transparenz weicht bei Pigmenten mit Ilmenitschicht der für Metalleffektpigmente charakteristischen Opazität und der scheinbar aus der Tiefe kommende weiche Glanz wird durch den harten Metallglanz ersetzt.

Auch der Metalleffektpigmente kennzeichnende, insbesondere bei Aluminiumeffektpigmenten ausgeprägte, Hell/ Dunkelflop kann bei den silberfarbenen ilmenitbeschichteten Pigmenten verstärkt beobachtet werden. Mithin ist bevorzugt, dass die silberfarbenen Pigmente nicht transparent sind und vorzugsweise einen metallischen Hell-Dunkel-Flop aufweisen.

Da die silberfarbenen Pigmente durch die Ilmenitschicht zwar ein metallisches Aussehen aufweisen, aber weder einen metallischen Kern noch eine metallische Schicht auf dem nichtmetallischen plättchenförmigen synthetischen Substrat vorhanden ist, bleiben die hervorragende Chemikalienstabilität und die hohe Temperaturbeständigkeit, durch die sich Perlglanzpigmente auszeichnen, erhalten. Selbstverständlich sind auch chemikalienstabile und temperaturbeständige Metalleffektpigmente kommerziell verfügbar, jedoch müssen diese, im Unterschied zu Perlglanzpigmenten, hierfür einer aufwendigen Nachbehandlung unterzogen werden.

In der erfindungsgemäßen kosmetischen Formulierung wird eine neue Pigmentkategorie eingesetzt, die in Bezug auf die chemische und mechanische Stabilität den Perlglanzpigmenten ähnlich ist, jedoch in ihren optischen Eigenschaften Metalleffektpigmenten täuschend ähnlich ist. Die in der erfindungsgemäßen kosmetischen Formulierung einzusetzenden silberfarbenen Pigmente sind in DE 10 2011 001 579 beschrieben, die hiermit unter Bezugnahme aufgenommen wird.

Die in der erfindungsgemäßen kosmetischen Formulierung einzusetzenden silberfarbenen Pigmente haben sich als äußerst temperatur- sowie korrosions- und chemikalienstabil erwiesen.

Die Chemikalienstabilität der silberfarbenen Pigmente wird anhand von Lackapplikationen auf Blechen überprüft, welche der Einwirkung einer Säure oder Lauge ausgesetzt sind.

Die Korrosionsbeständigkeit der silberfarbenen Pigmente wurde anhand ihres Gasungsverhaltens in einem wässrigen Carbomergel-System bestimmt.

Ob die in der erfindungsgemäßen kosmetischen Formulierung einzusetzenden silberfarbenen Pigmente über die gewünschte Temperaturbeständigkeit verfügen, wird durch Lagerung der Pigmente bei Temperaturen von 100°C bis 200°C festgestellt. Die Pigmente werden nach der Lagerung anhand von Rakelabzügen auf eventuelle Farbveränderungen untersucht.

Die farbneutralen silberfarbenen Pigmente mit metallischem Aussehen können basierend auf nichtmetallischen plättchenförmigen synthetischen Substraten mit oben genannten Eigenschaften überraschenderweise bereits in Anwesenheit einer sehr dünnen Ilmenitschicht mit einer mittleren Schichtdicke aus einem Bereich von 1 bis 20 nm, vorzugsweise 6 bis 15 nm, erhalten werden. Überraschenderweise ist die Ausbildung einer dickeren Ilmenitschicht nicht erforderlich. Mithin reicht die Ausbildung einer sehr dünnen Ilmenitschicht mit weniger als 20 nm, um ein silberfarbenes Pigment zu erhalten, das in seinen optischen Eigenschaften einem Metalleffektpigment, insbesondere einem Aluminiumeffektpigment, ähnlich ist.

In Abwesenheit weiterer als zur Ilmenitbildung notwendigen Eisenkomponenten kann ein Eisen/ Titan-Gewichtsverhältnis, berechnet als Verhältnis von elementarem Eisen zu elementarem Titan, aus einem Bereich von 0,1 bis 0,25 ausreichen, um die charakteristische Perlglanzoptik zu unterdrücken. Die silberfarbenen Pigmente gleichen nach Ilmenitbeschichtung in ihren optischen Eigenschaften Metalleffektpigmenten, wobei funktionelle Eigenschaften von Perlglanzpigmenten beibehalten werden. Somit können die silberfarbenen Pigmente der vorliegenden Erfindung idealerweise in Anwendungen eingesetzt werden, in denen zwar metallisches Aussehen, aber kein Metalleffektpigment gewünscht ist.

Ein Eisen/ Titan-Gewichtsverhältnis, berechnet als Verhältnis von elementarem Eisen zu elementarem Titan, von weniger als 0,1 würde die Deckfähigkeit von silberfarbenen Pigmenten verschlechtern, während ein Eisen/ Titan-Gewichtsverhältnis von über 0,25 nahezu keinen zusätzlichen Beitrag zur Deckfähigkeit liefert.

Zur Bestimmung des Eisen/ Titan-Gewichtsverhältnisses wird der aus Röntgenfluoreszenzmessungen bestimmte Titanoxidgehalt auf elementares Titan umgerechnet. Ebenfalls wird der Gehalt an Eisenverbindungen auf elementares Eisen umgerechnet. Wie bereits bei der Definition des Eisengehalts erwähnt, beschreibt auch der Titangehalt die Gesamtheit aller nachweisbaren Titanverbindungen im Pigment umgerechnet auf elementares Titan.

Das Eisen/ Titan-Gewichtsverhältnis der silberfarbenen Pigmente ist von der Teilchengröße des Pigmentes und/ oder der mittleren Dicke des nichtmetallischen plättchenförmigen synthetischen Substrates abhängig. Sowohl der Eisengehalt als auch der Titangehalt hängt mithin von der mittleren Teilchengröße $D_{50}$ und der mittleren Dicke der zu beschichtenden nichtmetallischen plättchenförmigen synthetischen Substrate ab. Die optische Schichtdicke der das plättchenförmige nichtmetallische synthetische Substrat umgebenden Schicht ist verantwortlich für die Farbe der resultierenden Pigmente.

Eine Beschichtung mit Titandioxid mit einer optischen Schichtdicke von 140 nm ergibt beispielsweise silberfarbene Perlglanzpigmente. Jedoch ist die zum Erreichen dieser optischen Schichtdicke erforderliche Menge an beispielsweise Titandioxid abhängig von mittlerer Teilchengröße $D_{50}$ und mittlerer Dicke der zu beschichtenden nichtmetallischen plättchenförmigen Substrate. Ein silberfarbenes, auf natürlichem Glimmer basierendes, Perlglanzpigment mit einer mittleren Teilchengröße $D_{50}$ von ungefähr 20 $\mu$m (z.B. Pearlescent Pigment Prestige Silver Star, Fa. Sudarshan Chemical Industries Limited, Indien), weist einen Titandioxidgehalt von ungefähr 30 Gew.-% auf, während ein entsprechendes Perlglanzpigment bei einer mittleren Teilchengröße $D_{50}$ von ungefähr 10 $\mu$m (z. B. Pearlescent Pigment Prestige Silk Silver Star, Fa. Sudarshan Chemical Industries Limited, Indien) einen Titandioxidgehalt von ungefähr 37 Gew.-% aufweist.

Um bei den silberfarbenen Pigmenten ein von mittlerer Teilchengröße $D_{50}$ und/ oder mittlerer Dicke des nichtmetallischen plättchenförmigen synthetischen Substrats unabhängiges Eisen/ Titan-Gewichtsverhältnis zu definieren, wird bei Bestimmung des Eisen/ Titan-Gewichtsverhältnis der Anteil der Beschichtung gemäß Formel (I) berücksichtigt:

$$\frac{\text{Eisengehalt (Gew.-\%)}}{\text{Titangehalt (Gew.-\%)}} \cdot \text{Anteil der Beschichtung (Gew.-\%)} \qquad (\text{I}).$$

Der Anteil der Beschichtung (Gew.-%) definiert sich aus dem Gesamtgewicht des Pigments abzüglich des Anteils des Substrates (Gew.-%). Der Eisengehalt ist definiert als die Gesamtheit aller nachweisbaren Eisenverbindungen im Pigment umgerechnet auf elementares Eisen. Ebenso ist der Titangehalt definiert als die Gesamtheit aller nachweisbaren Titanverbindungen im Pigment umgerechnet auf elementares Titan.

Das Eisen/ Titan-Gewichtsverhältnis gemäß Formel (I) der silberfarbenen Pigmente liegt vorzugsweise in einem Bereich von 1 bis 8, bevorzugt in einem Bereich von 2 bis 7,5, besonders bevorzugt in einem Bereich von 2,5 bis 7 und ganz besonders bevorzugt in einem Bereich von 3 bis 6.

Die Deckfähigkeit der silberfarbenen Pigmente wurde anhand der Helligkeitswerte L*, gemessen mit dem Gerät Byk mac, Fa. Byk-Gardner, von Lackapplikationen auf Schwarz-Weiß-Deckungskarten (Byko-Chart 2853, Fa. Byk-Gardner) bestimmt. Hierzu wurden die Helligkeitswerte auf dem schwarzen und weißen Hintergrund der Schwarz-Weiß-Deckungskarte bei einer Messgeometrie von 110°, relativ zum Ausfallwinkel des bei 45° eingestrahlten Lichts, bestimmt und deren Quotient gebildet. Im Rahmen dieser Erfindung werden Werte aus $L^*_{110,\ schwarz}/\ L^*_{110,\ weiß}$ von über 0,5 als deckend angesehen.

Die Deckfähigkeit der silberfarbenen Pigmente hängt weiterhin von deren Gesamtdicke ab. Je dicker das Substrat der silberfarbenen Pigmente ist, desto geringer ist deren Deckfähigkeit. Beispielsweise zeigen silberfarbene Pigmente, welche auf Glasplättchen mit einer Dicke von über 1 $\mu$m basieren, eine geringere Deckfähigkeit als silberfarbene Pigmente, welche plättchenförmigen synthetischen Glimmer mit einer Dicke von 400 nm als Substrat besitzen. Dies ist dadurch erklärbar, dass in einer definierten Menge an Pigment, von beispielsweise 1 g Pigment, die Anzahl einzelner Pigmente bei dünneren selbstverständlich größer ist, als dies bei dickeren Pigmenten der Fall wäre. Diese geringere Anzahl an Pigment ist in einer Applikation für eine vergleichsweise geringere Deckfähigkeit verantwortlich.

Im Unterschied zu transparenten Perlglanzpigmenten zeichnen sich opake Metalleffektpigmente durch ein höheres Deckvermögen aus. Das Deckvermögen der silberfarbenen Pigmente ist vergleichbar dem von Metalleffektpigmenten, insbesondere Aluminiumeffektpigmenten.

Metalleffektpigmente zeigen im Glanzwinkel den typischen Metallglanz, welcher außerhalb des Glanzwinkels verloren geht. Außerhalb des Glanzwinkels erscheinen Metalleffektpigmente enthaltende Applikationen weniger glänzend und dunkel. Dieser Effekt ist auch bei den silberfarbenen Pigmenten zu beobachten.

Ein Lack, welcher die silberfarbenen Pigmente enthält, zeigt nach der Applikation auf beispielsweise einem Blech und Trocknung einen im Wesentlichen winkelabhängigen Glanzeffekt bzw. einen sogenannten Hell-/ Dunkelflop. Diese Helligkeitsänderung wird durch den Flopindex beschrieben. Der Flopindex wird nach Alman folgendermaßen definiert (S. Schellenberger, M. Entenmann, A. Hennemann, P. Thometzek, Farbe und Lack, 04/2007, S. 130):

$$Flopindex = 2{,}69 \cdot (L_{E1} - L_{E3})^{1{,}11}/\ L_{E2}^{\ 0{,}86}$$

wobei $L_{E1}$ die Helligkeit des glanznahen Messwinkels (E1 = 15° zum Glanzwinkel), $L_{E2}$ die Helligkeit des Messwinkels zwischen glanznahem und glanzfernen Winkel (E2 = 45° zum Glanzwinkel) und $L_{E3}$ die Helligkeit des glanzfernen Messwinkels (E3 = 110° zum Glanzwinkel) darstellt. Je größer der Zahlenwert des Flopindexes ist, desto stärker kommt der Hell-/ Dunkelflop zum Ausdruck.

Der Flopindex der silberfarbenen Pigmente ist bei vergleichbarer Teilchengrößenverteilung und insbesondere bei einer vergleichbaren mittleren Teilchengröße $D_{50}$ dem eines Aluminiumeffektpigments nahezu identisch.

Der Quotient aus Flopindex zu $D_{50}$ beschreibt die winkelabhängige Helligkeitsänderung der silberfarbenen Pigmente in Abhängigkeit von der mittleren Teilchengröße $D_{50}$ des jeweiligen Pigmentes. Bevorzugt liegt der Quotient Flopindex/ $D_{50}$ in einem Bereich von 0,5 bis 1,9, besonders bevorzugt in einem Bereich von 0,6 bis 1,8 und ganz besonders bevorzugt in einem Bereich von 0,7 bis 1,7.

Eine Nachstellung des optischen Erscheinungsbildes der Pigmente ist durch einfaches Mischen eines herkömmlichen silberfarbenen Perlglanzpigments mit diversen Farbstoffen/-pigmenten wie beispielsweise Carbon Black nicht erreichbar. Bei der Verwendung von deckenden Farbstoffen/-pigmenten geht der Glanz sowie der Effekt des silberfarbenen Perlglanzpigmentes verloren. Bei der Verwendung von transparenten Farbstoffen/-pigmenten kann mithin keine Deckung erzielt werden.

Vergleicht man Lackapplikationen, welche die in der erfindungsgemäßen kosmetischen Formulierung einzusetzenden silberfarbenen Pigmente enthalten, mit Lackapplikationen, welche handelübliche silberfarbene Perlglanzpigmente enthalten, so ist der unterschiedliche optische Eindruck für einen Betrachter sofort erkennbar.

Lackapplikationen, welche ausschließlich die silberfarbenen Pigmente enthalten, erzeugen einen optischen Eindruck von reinem bzw. farbneutralem Silber, d.h. ohne zusätzliche Farbeindrücke. Des Weiteren zeigen diese Lackapplikationen ein metallisches Erscheinungsbild und einen außergewöhnlichen Glitzereffekt.

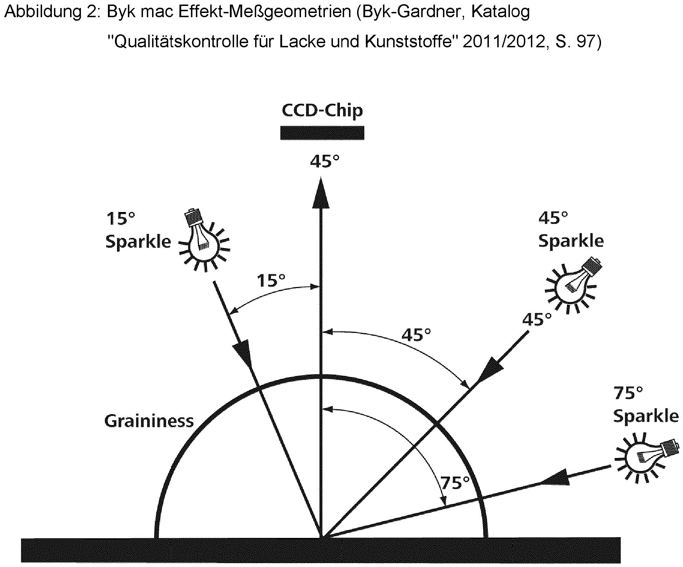

Um den optischen Effekt der silberfarbenen Pigmente objektiv zu beschreiben, wurden Mehrwinkelfarb- und Effektmessungen mit einem BYK-mac (Fa. Byk-Gardner) anhand von Lackapplikationen auf Blechen durchgeführt. Der BYK-mac misst den Gesamtfarbeindruck unter verschiedenen Beobachtungswinkeln und Lichtverhältnissen. Die Mehrwinkelfarbmessung dient hierbei zur Erfassung und Beschreibung des Hell-/ Dunkelflops und/ oder Farbflops von mit Effektpigmenten versehenen Lacken. Relativ zum Ausfallwinkel des bei 45° eingestrahlten Lichts werden die Messgeometrien (-15 °), + 15°, 25°, 45°, 75°, 110° gemessen. Zur Simulation von Effektänderungen bei direkter und diffuser Beleuchtung werden gleichzeitig Glitzereffekt und Körnigkeit mit Hilfe einer hochauflösenden CCD-Kamera kontrolliert. Der Glitzereffekt, verursacht durch das Reflexionsvermögen der einzelnen Effektpigmente, wird nur bei direkter Sonneneinstrahlung wahrgenommen und verändert sich in Abhängigkeit des Beleuchtungswinkels. Aus diesem Grund beleuchtet der Byk-mac die Probe mit sehr hellen LEDs unter drei verschiedenen Winkeln (15°/45°/75°, Abbildung 2). Die CCD-Kamera nimmt dabei senkrecht zur Oberfläche jeweils ein Bild auf. Die Bilder werden mit Hilfe von Bildverarbeitungsalgorithmen analysiert, wobei das Histogramm der Helligkeitsstufen als Basis für die Berechnung der Glitzerparameter verwendet wird. Um eine bessere Differenzierung zu gewährleisten, kann der Glitzereffekt durch ein zweidimensionales System beschrieben werden, der Glitzerfläche $S\_a$ und der Glitzerintensität $S\_i$, welche auch in einem eindimensionalen Wert, dem Glitzergrad $S\_G$ zusammengefasst werden können (Byk-Gardner, Katalog "Qualitätskontrolle für Lacke und Kunststoffe" 2011/2012, S. 97/ 98).

Die gemessene Glitzerfläche und Glitzerintensität wird durch die Orientierung der Pigmente beeinflusst. Ein gut, d.h. weitgehend planparallel zum Untergrund ausgerichtetes Pigment weist bei einem Vergleich der in den Beleuchtungsgeometrien 15°, 45° und 75° erhaltenen Glitzermesswerte $S\_a$, $S\_i$ und $S\_G$ die höchsten Messwerte bei einer Beleuchtungsgeometrie von 15° auf, da ein großer Teil der Pigmente das eingestrahlte Licht direkt reflektiert. Bei einer Beleuchtungsgeometrie von 45° wird das eingestrahlte Licht weitgehend direkt reflektiert und damit bei Beobachtung senkrecht zur Applikation als schwächerer Glitzereffekt wahrgenommen. Der bei dieser Beleuchtungsgeometrie beobachtete Glitzereffekt geht teilweise auf fehl-, d.h. nicht planparallel, orientierte Pigmente zurück, die das bei 45° eingestrahlte Licht in Richtung des Detektors ablenken können. Bei einer Beleuchtungsgeometrie von 75° wird senkrecht zur Applikation kein bzw. nur ein schwacher Glitzereffekt wahrgenommen. Dieser Effekt wird wiederum durch fehlgeordnete Pigmente bedingt.

Somit weist ein gut orientiertes Pigment den größten Glitzereffekt bei 15° auf, der minimale Glitzereffekt relativ zu der 15° Messung wird bei 75° beobachtet. Im Falle eines schlecht orientierten Pigments werden die Differenzen der bei 15°, 45° und 75° Beleuchtungsgeometrie beobachteten Messwerte kleiner, da durch die Fehlorientierung stets Licht in Richtung des Detektors reflektiert wird.

Maßgeblich für den optischen Eindruck ist der eindimensionale Glitzergrad $S\_G$. Je höher der Zahlenwert von $S\_G$, desto höher ist der auch vom Auge wahrnehmbare Glitzereffekt. In einer zweidimensionalen Darstellung kann der Glitzergrad $S\_G$ in die Komponenten Glitzerintensität $S\_i$ und Glitzerfläche $S\_a$ aufgeteilt werden. Da beide Komponenten einen maßgeblichen Einfluss auf den Glitzergrad $S\_G$ haben, kann es vorkommen, dass ein Pigment in den Messgeometrien 15°, 45° und 75° nahezu den gleichen Glitzergrad $S\_G$ aufweist, obwohl sich die Zahlenwerte von $S\_a$ und $S\_G$ in den betrachteten Winkeln deutlich erhöhen bzw. erniedrigen.

Im Unterschied zu beispielsweise auf natürlichem Glimmer basierenden silberfarbenen Perlglanzpigmenten, welche weder in Schichtaufbau noch Teilchengröße von den in der erfindungsgemäßen kosmetischen Formulierung einzusetzenden silberfarbenen Pigmenten verschieden sind, zeigen diese silberfarbenen Pigmente bei einer Messgeometrie von 15° weitaus höhere Werte für Glitzerintensität $S\_i$ und Glitzerfläche $S\_a$. Somit kann der für einen Betrachter sichtbare optische Unterschied auch messtechnisch belegt werden.

Bei einer mittleren Teilchengröße $D_{50}$ der silberfarbenen Pigmente aus einem Bereich von 15 bis 25 $\mu$m liegt die Glitzerintensität $S\_i$ bei einer Messgeometrie von 15°, relativ zum Ausfallwinkel des bei 45° eingestrahlten Lichts, vorzugsweise bei > 10, besonders bevorzugt bei > 11 und ganz besonders bevorzugt bei > 12.
Bei einer mittleren Teilchengröße $D_{50}$ der silberfarbenen Pigmente aus einem Bereich von 5 bis <15 $\mu$m liegt die Glitzerintensität $S\_i$ bei einer Messgeometrie von 15°, relativ zum Ausfallwinkel des bei 45° eingestrahlten Lichts, vorzugsweise bei > 5, besonders bevorzugt bei > 6 und ganz besonders bevorzugt bei > 7.

Neben der Glitzerintensität $S\_i$ ist, wie bereits erwähnt, auch der Flopindex von der mittleren Teilchengröße $D_{50}$ abhängig. Eine Änderung der mittleren Teilchengröße $D_{50}$ wirkt sich in besonderem Maße auf diese beiden Kennzahlen aus. Das Produkt aus Flopindex und Glitzerintensität $S\_i$ in Abhängigkeit von der mittleren Teilchengröße $D_{50}$ gemäß Formel (II)

$$Flopintensität(F_i) = \frac{Flopindex \cdot S\_i}{D_{50}} \qquad (II)$$

wird als Flopintensität ($F_i$) definiert und belegt eindrucksvoll den visuell sichtbaren Unterschied der erfindungsgemäßen silberfarbenen Pigmente zu kommerziell verfügbaren Pigmenten. Bevorzugt liegt der Wert für das Produkt aus Flopindex und Glitzerintensität in Abhängigkeit von der mittleren Teilchengröße $D_{50}$ gemäß Formel (II) bei wenigstens 10. Je höher dieser Wert ist, desto glitzernder, metallischer erscheint das Pigment einem Betrachter.

Zur Herstellung der silberfarbenen Pigmente wird das nichtmetallische plättchenförmige synthetische Substrat vorzugsweise in Wasser suspendiert. Der Suspension wird vorzugsweise bei einer Temperatur aus einem Bereich von 50°C bis 100°C und vorzugsweise bei einem konstant gehaltenen pH-Wert aus einem Bereich von 1,4 bis 4,0 vorzugsweise eine wasserlösliche anorganische Zinn- und anschließend vorzugsweise eine wasserlösliche anorganische Titanverbindung zugegeben. Nach beendeter Zugabe der wasserlöslichen Titanverbindung wird die nun erhaltene Suspension vorzugsweise wenigstens 30 Minuten nachgerührt und anschließend vorzugsweise eine wasserlösliche anorganische Eisenverbindung zugegeben. Das nun mit einer Titanoxidhydrat-/ Titanhydroxidschicht und einer Eisenoxidhydrat-/ Eisenhydroxidschicht belegte nichtmetallische plättchenförmige synthetische Substrat wird nach beendeter Reaktion abgetrennt, gegebenenfalls gewaschen, optional getrocknet und vorzugsweise bei Temperaturen im Bereich von 500°C bis 1200°C unter reduzierenden Bedingungen, bevorzugt in Gegenwart von Formiergas ($N_2$/ $H_2$) thermisch behandelt bzw. kalziniert. Die thermische Behandlung bzw. Kalzinierung wird vorzugsweise solange durchgeführt, bis das vorhandene Eisenoxidhydrat/ Eisenhydroxid nahezu vollständig, vorzugsweise vollständig zu Ilmenit umgesetzt ist.

Gemäß WO 2004/099319 A2 ist es von außerordentlicher Bedeutung, dass zur Herstellung ilmenithaltiger Pigmente, die wasserlösliche anorganische Titan- und die wasserlösliche anorganische Eisenverbindung gleichzeitig auf einem plättchenförmigen, gegebenenfalls bereits beschichteten Substrat aufgebracht werden. Es wird weiterhin darauf hingewiesen, dass durch die gleichzeitige Zugabe beider Komponenten Pigmente mit gegenüber dem Stand der Technik verbesserten optischen Eigenschaften erhalten werden können.

Im Rahmen dieser Erfindung wurde jedoch überraschenderweise festgestellt, dass bei Verwendung nichtmetallischer plättchenförmiger synthetischer Substrate auch bei einer, wie aus dem Stand der Technik bekannten, nacheinander erfolgenden Zugabe der jeweils wasserlöslichen anorganischen Titan- und Eisenverbindung hochglänzende silberfarbene Pigmente mit hoher Glitzerintensität, ausgeprägtem Hell/Dunkelflop und hoher Deckkraft erhalten werden können, welche nicht die in WO 2004/099319 A2 erwähnten Nachteile aufweisen.

Weiterhin hat es sich als äußerst vorteilhaft erwiesen, im Unterschied zu beispielsweise EP 0 246 523 A2 die wasserlösliche anorganische Eisenverbindung *in situ* zuzugeben und kein bereits kalziniertes, mit Titandioxid beschichtetes, Perlglanzpigment, als Ausgangsmaterial zu verwenden. Bei identischem Schichtaufbau, bestehend aus beispielsweise einer Titandioxidschicht sowie einer ilmenithaltigen Schicht, macht sich der verfahrenstechnische Unterschied, ob vor Aufbringung der wasserlöslichen anorganischen Eisenverbindung kalziniert wurde oder *in situ* weiterbeschichtet wurde, in der Chemikalienstabilität bemerkbar. Die silberfarbenen Pigmente, welche ohne vorherige Kalzination hergestellt werden, sind deutlich stabiler gegenüber Säure bzw. Lauge als gemäß EP 0 246 523 A2 hergestellte Perlglanzpigmente, welche von einem kalzinierten Pigment ausgehen. Auch gegenüber Perlglanzpigmenten, welche durch eine gleichzeitige Zugabe einer jeweils wasserlöslichen anorganischen Titan- und Eisenverbindung erhalten wurden, sind die silberfarbenen Pigmente hinsichtlich der Chemikalienstabilität überlegen. Die Aufbringung der Eisenoxidhydrat-/ Eisenhydroxidschicht auf die nichtkalzinierte bzw. ungeglühte Titanoxidhydrat-/ Titanhydroxidschicht ist wesentlich für den strukturellen Unterschied der Pigmente gegenüber den aus dem Stand der Technik bekannten Perlglanzpigmenten. Die Erfinder gehen davon aus, dass bei der Verwendung von unkalzinierten Titanoxidhydrat-/ Titanhydroxidschichten die nachfolgend aufgebrachte Eisenoxidhydrat-/ Eisenhydroxidschicht stärker in die Poren der angrenzenden Titanoxidhydrat-/ Titanhydroxidschicht eindringen kann. Die bessere Durchdringung bewirkt eine nahezu vollständige Umwandlung zu Ilmenit, nicht nur direkt an der Grenzfläche.

Bei der Verwendung von bereits kalzinierten, mit Titandioxid belegten, Perlglanzpigmenten ist eine derartige Durchdringung nicht möglich. Außerdem ist kalziniertes Titandioxid deutlich reaktionsträger als Titanoxidhydrat oder Titanhydroxid. Die Umwandlung zu Ilmenit ist daher selbst bei geringen Schichtdicken zumeist unvollständig. Dies äußert sich insbesondere in einer schlechteren Chemikalienstabilität.

Bei den in der erfindungsgemäßen kosmetischen Formulierung zu verwendenden silberfarbenen Pigmenten handelt es sich nicht um Mehrschichtperlglanzpigmente, deren Schichtaufbau eine hochbrechende, niedrigbrechende, hochbrechende Beschichtung umfasst.

Für die Nachahmung der optischen Eigenschaften eines Aluminiumeffektpigments sollte das nichtmetallische plättchenförmige synthetische Substrat vorzugsweise nachstehende Eigenschaften aufweisen. Bei einer bevorzugten Variante der Erfindung erfüllt das nichtmetallische plättchenförmige synthetische Substrat sämtliche nachstehend genannten Eigenschaften bzgl. Fremdionengehalt, Substratdicke und Helligkeit.

Um neben dem silbernen Farbeindruck bei den Pigmenten einen Farbstich oder eine Farbgebung zu vermeiden, sollte das nichtmetallische plättchenförmige synthetische Substrat nur vernachlässigbare Mengen an eingelagerten Fremdionen, die den Farbton verändern können, aufweisen. Vorzugsweise ist das nichtmetallische plättchenförmige synthetische Substrat im Wesentlichen farblos, vorzugsweise farblos.

Die silberfarbenen Pigmente sind vorzugsweise nicht bzw. nur schwach magnetisch. Weiterhin sind die silberfarbenen Pigmente nicht elektrisch leitfähig.

Die mittlere Dicke des Substrats wird vorzugsweise so gewählt, dass die in der erfindungsgemäßen kosmetischen Formulierung einzusetzenden silberfarbenen Pigmente eine hohe Deckfähigkeit aufweisen. Die mittlere Dicke der zu beschichtenden nichtmetallischen plättchenförmigen synthetischen Substrate liegt vorzugsweise in einem Bereich von 50 nm bis 5000 nm, bevorzugt in einem Bereich von 60 nm bis 3000 nm und besonders bevorzugt in einem Bereich von 70 nm bis 2000 nm.

Bei einer Ausführungsform liegt die mittlere Dicke für Glasplättchen als zu beschichtendes Substrat vorzugsweise in einem Bereich von 750 nm bis 1500 nm. Derartige Glasplättchen sind kommerziell auf breiter Basis verfügbar. Weitere Vorteile bieten dünnere Glasplättchen. Dünnere Substrate führen zu einer geringeren Gesamtschichtdicke der silberfarbenen Pigmente. So sind ebenfalls bevorzugt Glasplättchen, deren mittlere Dicke in einem Bereich von 100 nm bis 700 nm, weiter bevorzugt in einem Bereich von 150 nm bis 600 nm, besonders bevorzugt in einem Bereich von 170 nm bis 500 nm und ganz besonders bevorzugt in einem Bereich 200 nm bis 400 nm liegt.

Bei einer weiteren Ausführungsform liegt die mittlere Dicke für synthetischen Glimmer als zu beschichtendes nichtmetallisches plättchenförmiges Substrat bevorzugt in einem Bereich von 100 nm bis 700 nm, weiter bevorzugt in einem Bereich von 120 nm bis 600 nm, besonders bevorzugt in einem Bereich von 140 nm bis 500 nm und ganz besonders bevorzugt in einem Bereich von 150 nm bis 450 nm.

Beschichtet man nichtmetallische plättchenförmige synthetische Substrate unterhalb einer mittleren Dicke von 50 nm mit beispielsweise hochbrechenden Metalloxiden, so werden extrem bruchempfindliche Pigmente erhalten, die schon beim Einarbeiten in das Anwendungsmedium zerbrechen können, was wiederum eine signifikante Herabsetzung des Glanzes bedingt. Zudem dauern die Beschichtungszeiten dieser dünnen Substrate mit beispielsweise hochbrechenden Metalloxiden aufgrund der hohen spezifischen Oberflächen (Oberfläche pro Gewichtseinheit Pigment) dieser nichtmetallischen plättchenförmigen synthetischen Substrate sehr lange, was hohe Herstellkosten verursacht. Oberhalb einer mittleren Substratdicke von 5000 nm können die Pigmente insgesamt zu dick werden. Damit kann eine schlechtere spezifische Deckfähigkeit, d.h. abgedeckte Fläche pro Gewichtseinheit an Pigment, sowie eine geringere planparallele Orientierung im Anwendungsmedium verbunden sein. Aus einer schlechteren Orientierung wiederum resultiert ein verminderter Glanz.

Die mittlere Dicke des nichtmetallischen plättchenförmigen synthetischen Substrates wird anhand eines gehärteten Lackfilmes, in dem die Pigmente im Wesentlichen planparallel zum Untergrund ausgerichtet sind, bestimmt. Hierzu wird ein Querschliff des gehärteten Lackfilmes unter einem Rasterelektronenmikroskop (REM) untersucht, wobei die Dicke des nichtmetallischen plättchenförmigen synthetischen Substrates von 100 Pigmenten bestimmt und statistisch gemittelt wird.

Weiterhin ist es bevorzugt, dass das nichtmetallische plättchenförmige synthetische Substrat eine hohe Helligkeit, ausgedrückt als L*-Wert von wenigstens 90, weiter bevorzugt von wenigstens 92, noch weiter bevorzugt von wenigstens 95, aufweist. Die Helligkeit wird hierbei durch diffuse Farbmessung anhand von Pulverschüttungen bestimmt.

Die Oberfläche des nichtmetallischen plättchenförmigen synthetischen Substrats ist des Weiteren vorzugsweise sehr glatt und frei von Lufteinschlüssen, Sprüngen, Rissen und/ oder anderen eine Lichtstreuung verursachenden Bestandteilen.

Insbesondere haben sich synthetische Glimmerplättchen, welche die in Tabelle 1 aufgeführte Zusammensetzung innerhalb der genannten Grenzen umfassen, als sehr geeignetes nichtmetallisches Substrat für die Herstellung der in der erfindungsgemäßen kosmetischen Formulierung einzusetzenden silberfarbenen Pigmente mit metallischem Aussehen erwiesen.

Die silberfarbenen Pigmente können eine beliebige mittlere Teilchengröße $D_{50}$ aufweisen. Die $D_{50}$-Werte der silberfarbenen Pigmente liegen vorzugsweise in einem Bereich von 3 bis 80 $\mu$m. Bevorzugt weisen die silberfarbenen Pigmente einen $D_{50}$-Wert aus einem Bereich von 5 bis 63 $\mu$m, besonders bevorzugt aus einem Bereich von 7 bis 56 $\mu$m und ganz besonders bevorzugt aus einem Bereich von 9 bis 49 $\mu$m auf.

Die $D_{10}$-Werte der silberfarbenen Pigmente liegen vorzugsweise in einem Bereich von 1 bis 25 $\mu$m. Bevorzugt weisen die silberfarbenen Pigmente einen $D_{10}$-Wert aus einem Bereich von 2 bis 21 $\mu$m, besonders bevorzugt aus einem Bereich von 3 bis 18 $\mu$m und ganz besonders bevorzugt aus einem Bereich von 4 bis 14 $\mu$m auf.

Die $D_{90}$-Werte der silberfarbenen Pigmente liegen vorzugsweise in einem Bereich von 6 bis 250 $\mu$m. Bevorzugt weisen die silberfarbenen Pigmente einen $D_{90}$-Wert aus einem Bereich von 15 bis 210 $\mu$m auf.

Der $D_{10}$-, $D_{50}$- bzw. $D_{90}$-Wert der Summenhäufigkeitsverteilung der volumengemittelten Größenverteilungsfunktion, wie sie durch Laserbeugungsmethoden erhalten wird, gibt an, dass 10%, 50% bzw. 90% der silberfarbenen Pigmente einen Durchmesser aufweisen, der gleich oder kleiner als der jeweils angegebene Wert ist. Hierbei wird die Größenverteilungskurve der Pigmente mit einem Gerät der Fa. Malvern (Gerät: MALVERN Mastersizer 2000) gemäß Herstellerangaben bestimmt. Die Auswertung der Streulichtsignale erfolgte dabei nach der Fraunhofer Methode.

Die silberfarbenen Pigmente können optional mit wenigstens einer äußeren Schutzschicht versehen sein, die die Licht- und/ oder chemische Stabilität des Pigmentes weiter erhöht. Bei der äußeren Schutzschicht kann es sich auch um eine Nachbeschichtung handeln, die die Handhabung der silberfarbenen Pigmente bei Einarbeitung in unterschiedliche Medien erleichtert.

Die äußere Schutzschicht kann des Weiteren an der Oberfläche organischchemisch modifiziert sein. Beispielsweise können ein oder mehrere, vorzugsweise kosmetisch zugelassene, Silane auf dieser äußeren Schutzschicht aufgebracht sein. Bei den Silanen kann es sich um Alkylsilane mit verzweigten oder unverzweigten Alkylresten mit 1 bis 24 C-Atomen, vorzugsweise 6 bis 18 C-Atomen handeln.

Bei einer weiterhin bevorzugten Ausführungsform ist das Silan ohne funktionelle Bindungsgruppe ein Alkylsilan. Das Alkylsilan weist vorzugsweise die Formel $R_{(4-z)}Si(X)_z$ auf. Hierbei ist z eine ganze Zahl von 1 bis 3, R ist eine substituierte oder unsubstituierte, unverzweigte oder verzweigte Alkylkette mit 10 bis 22 C-Atomen und X steht für eine Halogen- und/ oder Alkoxygruppe. Bevorzugt sind Alkylsilane mit Alkylketten mit mindestens 12 C-Atomen. R kann auch zyklisch mit Si verbunden sein, wobei in diesem Fall z üblicherweise 2 ist.

Für die Einarbeitung von mit Silanen nachbeschichteten bzw. mit einer äußeren Schutzschicht versehenen Pigmenten in kosmetischen Formulierungen muss darauf geachtet werden, dass das entsprechende Silan bzw. das Material der äußeren Schutzschicht den länderspezifischen Anforderungen der Kosmetikverordnungen entspricht. Darüber hinaus muss ein INCI-Name verfügbar sein. Gängige Beispiele hierfür sind Organosilane mit den INCI-Namen: Triethoxycaprylylsilan, Stearyl Triethoxysilan, Polymethylsilsesquioxane/trimethylsiloxysilicate, Sodium Carboxyethylsilanetriol.

In einer weiteren Ausführungsform können die silberfarbenen Pigmente mit wenigstens einer organischen Nachbeschichtung, die in einer dem Fachmann bekannten Weise aufgebracht wird, belegt sein.

Bevorzugte Verfahren zur Nachbeschichtung umfassen beispielsweise:

- PEG-Siliconbeschichtung, wie beispielsweise die "AQ" Modifizierung, erhältlich von LCW,
- Chitosanbeschichtung, wie beispielsweise die "CTS" Modifizierung, erhältlich von LCW,
- Triethoxycaprylylsilanbeschichtung, wie beispielsweise die "AS" Modifizierung, erhältlich von LCW,
- Methiconbeschichtung, wie beispielsweise die "SI" Modifizierung, erhältlich von LCW,
- Dimethiconbeschichtung, wie beispielsweise die "Covasil 3.05" Modifizierung, erhältlich von LCW,
- Dimethicone/Trimethylsiloxysilicatbeschichtung, wie beispielsweise die "Covasil 4.05" Modifizierung, erhältlich von LCW,
- Lauroyllysinbeschichtung, wie beispielsweise die "LL" Modifizierung, erhältlich von LCW,
- Lauroyllysinmethiconbeschichtung, wie beispielsweise die "LL/SI" Modifizierung, erhältlich von LCW,
- Magnesiummyristatbehandlung, wie beispielsweise die "MM" Modifizierung, erhältlich von LCW,
- Aluminumdimyristatbeschichtung, wie beispielsweise die "MI" Modifizierung, erhältlich von Miyoshi,
- Perfluoropolymethylisopropyletherbeschichtung, wie beispielsweise die "FHC" Modifizierung, erhältlich von LCW,
- Dinatriumstearoylglutamatbeschichtung, wie beispielsweise die "NAI" Modifizierung, erhältlich von Miyoshi,
- Perfluoroalkylphosphatbehandlung, wie beispielsweise die "PF" Modifizierung, erhältlich von Daito,
- Acrylat/Dimethicon und Perfluoroalkylphosphatbeschichtung, wie beispielsweise die "FSA" Modifizierung, erhältlich von Daito,
- Polymethylhydrogensiloxan/Perfluoroalkylphosphatbeschichtung, wie beispielsweise die "FS01" Modifizierung, erhältlich von Daito,
- Lauryllysin/Aluminumtristearatbeschichtung, wie beispielsweise die "LL-StAl" Modifizierung, erhältlich von Daito,
- Octyltriethylsilanbeschichtung, wie beispielsweise die "OTS" Modifizierung, erhältlich von Daito,
- Octyltriethylsilan/Perfluoroalkylphosphatbeschichtung, wie beispielsweise die "FOTS" Modifizierung, erhältlich von Daito,
- Acrylatdimethiconcopolymerbeschichtung, wie beispielsweise die "ASC" Modifizierung, erhältlich von Daito,
- Isopropyltitantriisostearatbeschichtung, wie beispielsweise die "ITT" Modifizierung, erhältlich von Daito,
- Mikrokristalline Cellulose- und Carboxymethylcellulosebeschichtung, wie beispielsweise die "AC" Modifizierung, erhältlich von Daito,
- Acrylatcopolymerbeschichtung, wie beispielsweise die "APD" Modifizierung, erhältlich von Daito,
- Perfluoroalkylphosphat/Isopropyltitantriisostearatbeschichtung, wie beispielsweise die "PF+ITT" Modifizierung, erhältlich von Daito.

Über die Oberflächenmodifizierung kann beispielsweise Hydrophobie der Pigmentoberfläche verändert und/ oder eingestellt werden. In O/W-, W/O- und W/Si-Emulsionssystemen kann durch eine hydrophobe Oberflächenbelegung eine verbesserte Einarbeitbarkeit und eine längere Emulsionsstabilität erzielt werden.

Bei einer weiteren Ausführungsform umfasst die vorliegende Erfindung eine kosmetische Formulierung umfassend silberfarbene Pigmente basierend auf nichtmetallischen plättchenförmigen synthetischen Substraten, welche mit einer Titandioxid- sowie einer ilmenithaltigen Schicht belegt sind, bezogen auf das Gesamtgewicht der Pigmente einen Gehalt an Eisenverbindungen, berechnet als elementares Eisen, im Pigment von weniger als 5 Gew.-% und in Abhängigkeit von der Beschichtung ein Eisen/ Titan-Gewichtsverhältnis gemäß

$$\frac{\text{Eisengehalt (Gew.-\%)}}{\text{Titangehalt (Gew.-\%)}} \cdot \text{Anteil der Beschichtung (Gew.-\%)} \qquad (I)$$

aus einem Bereich von 1 bis 8 aufweisen.

Bei einer weiteren Ausführungsform umfasst die Erfindung eine kosmetische Formulierung umfassend silberfarbene Pigmente basierend auf synthetischen Glimmerplättchen, welche nach Belegung mit einer wasserlöslichen Zinnverbindung, einer wasserlöslichen Titanverbindung sowie *in situ* einer wasserlöslichen Eisenverbindung nach Kalzination unter reduzierenden Bedingungen erhalten werden, wobei die Pigmente durch ihren farbneutralen Silberfarbton und geringe Chromawerte bei einer Messgeometrie von 110°, relativ zum Ausfallwinkel des bei 45° eingestrahlten Lichts, von $C^*_{110} \leq 2,4$, gemessen anhand von Lackapplikationen auf Blechen, gekennzeichnet sind.

Bei einer weiteren Ausführungsform umfasst die Erfindung eine kosmetische Formulierung umfassend silberfarbene Pigmente basierend auf synthetischen Glimmerplättchen mit einer Helligkeit $L^*$ von über 90, vorzugsweise über 92, weiter bevorzugt über 95, welche einem Lacksystem nach Applikation und Trocknung einen außergewöhnlichen starken Glitzereffekt verleihen.

Bei einer weiteren Ausführungsform umfasst die Erfindung eine kosmetische Formulierung umfassend silberfarbene Pigmente, welche in ihrem optischen Erscheinungsbild nicht oder nur unwesentlich von Metalleffektpigmenten zu unterscheiden sind und deren Flopindex in Abhängigkeit von der mittleren Teilchengröße $D_{50}$ nahezu identisch dem von Aluminiumeffektpigmenten ist.

Bei einer bevorzugten Ausführungsform befindet sich die ilmenithaltige Schicht der silberfarbenen Pigmente im Schichtaufbau außen und ist optional mit wenigstens einer Schutzschicht umgeben. Bei einer besonders bevorzugten Ausführungsform umfassen die silberfarbenen Pigmente eine einzige Schicht aus Titandioxid in der Rutilmodifikation, eine einzige ilmenithaltige Schicht, optional wenigstens eine Schutzschicht, wobei zwischen der Titandioxid- und der ilmenithaltigen Schicht eine zumindest partielle Durchdringung erfolgt und ein Konzentrationsgradient zwischen beiden Schichten vorliegt.

In kosmetischen Formulierungen können die silberfarbenen Pigmente mit für die jeweilige Anwendung geeigneten Roh-, Hilfs- und Wirkstoffen kombiniert werden. Die Konzentration der silberfarbenen Pigmente in der Formulierung kann zwischen 0,001 Gew.-% für Rinse-off-Produkte und 40,0 Gew.-% für Leave-on-Produkte, jeweils bezogen auf das Gesamtgewicht der Formulierung, liegen.

Die silberfarbenen Pigmente eignen sich insbesondere für die Anwendung in Kosmetika, wie z.B. Körperpuder, Gesichtspuder, gepresstem und losem Puder, Pudercreme, Augenmakeup wie Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haargel, Haarwachs, Haarmascara, permanente oder semi-permanente Haarfarben, temporäre Haarfarben, Hautpflegekompositionen wie Lotions, Gele, Emulsionen sowie Nagellackkompositionen.

Die silberfarbenen Pigmente lassen sich auch vorteilhaft in Abmischungen mit transparenten und deckenden Weiß-, Bunt- und Schwarzpigmenten sowie weiteren Effektpigmenten in der erfindungsgemäßen kosmetischen Formulierung einsetzen.

Zur Erzielung spezieller Farbeffekte können in den Kosmetikapplikationen neben den silberfarbenen Pigmenten weitere Farbmittel und/ oder herkömmliche Effektpigmente und/ oder Mischungen hiervon in variablen Mengenverhältnissen eingesetzt werden. Als herkömmliche Effektpigmente können beispielsweise handelsübliche Perlglanzpigmente auf der Basis von mit hochbrechenden Metalloxiden beschichteten natürlichen Glimmerplättchen (wie z.B. die Produktgruppe Prestige, Fa. Sudarshan Chemical Industries Limited, Indien), BiOCl-Plättchen, $TiO_2$-Plättchen, Perlglanzpigmente auf der Basis von mit hochbrechenden Metalloxiden beschichteten synthetischen Glimmerplättchen (wie z.B. die Produktgruppe SynCrystal, Fa. Eckart) oder basierend auf mit hochbrechenden Metalloxiden beschichteten Glasplättchen (wie z.B. die Produktgruppe MIRAGE, Fa. Eckart), basierend auf mit hochbrechenden Metalloxiden beschichteten $Al_2O_3$- oder $SiO_2$-Plättchen oder basierend auf mit hoch-und/ oder niedrigbrechenden Metalloxiden beschichteten BiOCl- oder $TiO_2$-Plättchen verwendet werden. Außerdem können auch Metalleffektpigmente, wie z.B. die Produktgruppe Visionaire, Fa. Eckart, zugegeben werden. Die Farbmittel können aus anorganischen oder organischen Pigmenten ausgewählt sein.

Die erfindungsgemäße kosmetische Formulierung kann neben dem silberfarbenen Pigment und gegebenenfalls weiteren farb- und/ oder effektgebenden Komponenten wenigstens ein mikronisiertes Wachs umfassen. Das mikronisierte Wachs kann die haptischen Eigenschaften der erfindungsgemäßen kosmetischen Formulierung beeinflussen. Handelt es sich bei der erfindungsgemäßen kosmetischen Formulierung z.B. um einen Nagellack, so können durch die Zugabe wenigstens eines mikronisierten Wachses sowohl dessen optische als auch dessen haptische Eigenschaften beeinflusst werden. Der zu erzielende optische und haptische Effekt ist hierbei unter anderem abhängig der Teilchengröße des wenigstens einen mikronisierten Wachses.

Mikronisierte Wachse sind üblicherweise bei Raumtemperatur (25°C) fest und gehen oberhalb ihres Schmelzbereichs ohne Zersetzung vom festen in den flüssigen Zustand über. Die Konsistenz der mikronisierten Wachse kann von fest bis brüchig hart reichen. Mikronisierte Wachse können durchscheinend bis opak sein, sie können weiterhin zusätzlich mit einem kosmetisch zulässigen Farbmittel eingefärbt werden. Die mikronisierten Wachse können bereits bei einer Temperatur von 40°C zu schmelzen beginnen. Bevorzugt liegt der Schmelzbereich zwischen 51 und 399°C, weiter bevorzugt zwischen 71 und 389°C, besonders bevorzugt zwischen 91 und 379°C und insbesondere bevorzugt zwischen 111 und 369°C. Der Schmelzbereich der mikronisierten Wachse wird hierbei gemäß der DGF (Deutsche Gesellschaft für Fettwissenschaften) Einheitsmethode C-IV 3a mit einem Schmelzpunktmessgerät (Modell 5A 6797, Fa. Gallenkamp) bestimmt.

Als mikronisierte Wachse können natürliche und/ oder synthetische Wachse eingesetzt werden. Weiterhin können als mikronisierte Wachse vorzugsweise mit kosmetisch zugelassenen Farbmitteln eingefärbte natürliche und/ oder synthetische Wachse verwendet werden. Außerdem können auch Mischungen von jeweils unterschiedlichen gefärbten und/ oder ungefärbten mikronisierten Wachsen der erfindungsgemäßen kosmetischen Formulierung zugegeben werden.

Als natürliche mikronisierte Wachse können beispielsweise mikronisiertes Bienenwachs (INCI: Cera Alba Beeswax), mikronisiertes Candellilawachs (INCI: Candellila Cera) oder mikronisiertes Carnaubawachs (INCI: Copernicia Cerifera (Carnauba) Wax), kommerziell erhältlich z.B. als Carnauba Super-Micropowder (Fa. Kahl) oder Microcare 350 (Fa. Micro Powders) eingesetzt werden. Es können selbstverständlich auch verschiedene natürliche mikronisierte Wachse der erfindungsgemäßen kosmetischen Formulierung zugesetzt werden.

Ebenfalls zum Einsatz kommen können Gemische natürlicher und synthetischer mikronisierter Wachse. Hierzu zählen unter anderem mikronisiertes Carnauba-/ PE-Wachs (INCI: Carnauba/ Polyethylene), kommerziell erhältlich als z.B. Microcare 300, Microcare 310 (Fa. Micro Powders) oder mikronisiertes Carnauba-/synthetisches Wachs (INCI: Carnauba/ Synthetic Wax), kommerziell erhältlich als z.B. Microcare 325 (Fa. Micro Powders).

Auch bioabbaubare Biopolymere in mikronisierter Form wie z.B. Polymilchsäure (INCI: Polylactic Acid), kommerziell erhältlich z.B. als Ecosoft 608, Ecosoft 608X (Fa. Micro Powders) bzw. Ecosoft 611 (INCI: Polylactic Acid Copernecia Cerifera (Carnauba) Wax, Fa. Micro Powders) können in der erfindungsgemäßen kosmetischen Formulierung eingesetzt werden.

Als mikronisierte Wachse können unter anderem synthetische Wachse (INCI: (Oxidized) Synthetic Wax), kommerziell erhältlich z.B. als Microease 110XF, Microease 110S, Microease 114 S, Microease 116 (Fa. Micro Powders), mikronisierte PE-Wachse, kommerziell erhältlich z.B. als Ceraflour 990 oder Ceraflour 991 (Fa. BYK-Chemie), als Ceridust VP 3610 oder TP Ceridust 6050 M (Fa. Clariant), als PE Super Micropowder (INCI: Polyethylene, Fa. Kahl), als Micropoly 200, Micropoly 210, Micropoly 220, Micropoly 220L, Micropoly 250S (INCI: (Oxidized) Polyethylene, Fa. Micro Powders) oder als PE Microspheres (Fa. Cospheric), mikronisierte PP-Wachse, kommerziell erhältlich als Ceraflour 913, Ceraflour 914, Ceraflour 915, Ceraflour 916 (Fa. BYK-Chemie), Synafil W 1234 (INCI: Polypropylene, Fa. Eckart) oder als Mattewax 511 (INCI: Polypropylene, Fa. Micro Powders), mikronisierte PMMA Wachse (Polymethylmethacrylat, CAS: 9011-14-7), kommerziell erhältlich z.B. als PMMA Microspheres (Fa. Cospheric) oder mikroniersierte Polytetrafluorethylenwachse (INCI: PTFE), kommerziell erhältlich als z.B. PTFE-Super Micropowder (Fa. Kahl), Microslip 519, Microslip 519 L (Fa. Micro Powders) oder Ceridust 9202 F, Ceridust 9205 F (Fa. Clariant) eingesetzt werden.

Weiterhin können auch Gemische verschiedener mikronisierter synthetischer Wachse wie beispielsweise aus PE-/ Polytetrafluorethylen (INCI: Polyethylene/PTFE), kommerziell erhältlich z.B. als PE-PTFE Super Micropowder (Fa. Kahl), Microsilk 419 (Fa. Micro Powders) oder Ceridust 3920 F bzw. Ceridust 9325 F (Fa. Clariant), aus PE-/ Polytetrafluorethylen/ synthetischen Wachs (INCI: Polyethylene/PTFE/ Synthetic Wax), kommerziell erhältlich z.B. als Microsilk 418 (Fa. Micro Powders), aus PP-/Polytetrafluorethylen (INCI: Polypropylene/ PTFE), kommerziell erhältlich unter anderem als Microsilk 920 (Fa. Micro Powders) oder aus Polyethylen/ organischem Ester, wie Ceridust 3831 (Fa. Clariant) Verwendung finden.

Ein mikronisiertes Bistearylethylendiamidwachs, kommerziell erhältlich als Ceridust 3910 (Fa. Clariant) kann ebenfalls zum Einsatz kommen.

Auch mit Farbmitteln eingefärbte synthetische mikronisierte Wachse können der erfindungsgemäßen kosmetischen Formulierung zugegeben werden. Zu erwähnen sind hier unter anderem die gefärbten Microspheres, wie White, Black, Blue, Green, Orange, Pink, Purple, Red, Yellow oder Grey Microspheres (Fa. Cospheric). Alternativ oder zusätzlich können mit Titandioxid beschichtete Substrate, z.B. hohle Glaskügelchen, wie aus US 2011/0052804 A1 bekannt, zum Einsatz kommen. Die mikronisierten Wachse können der erfindungsgemäßen kosmetischen Formulierung gegebenenfalls auch als Dispersion zugegeben werden. Beispiele für kommerziell erhältliche Dispersionen (Fa. Micro Powders) sind Microspersion 220PC (INCI: Polyethylene, Isohexadecane), Microspersion 419PC (INCI: Polyethylene, PTFE, Isohexadecane), Microspersion 511PC (INCI: Polypropylene, Isodecane, Polyethylene) oder Microspersion 519PC (INCI: PTFE, Isodecane, Polyethylene).

Vorzugsweise werden der erfindungsgemäßen kosmetischen Formulierung ungefärbte synthetische mikronisierte Wachse zugegeben.

Bei einer weiteren Ausführungsform können auch Mischungen, die wenigstens ein mikronisiertes Wachs und wenigstens eine Sorte an hohlen Glaskügelchen enthalten, der erfindungsgemäßen kosmetischen Formulierung zugegeben werden. Als hohle Glaskügelchen können beispielsweise Hollow Glass Microspheres (Natriumsilikat, CAS Nr. 1344-09-8, Natriumborat CAS Nr. 7775-19-1, amorphes Siliziumdioxid CAS Nr. 7631-86-9; Fa. Cospheric) eingesetzt werden. Die mittlere Teilchengröße $D_{50}$ der hohlen Glaskügelchen liegt in einem Bereich von 27 bis 90 µm, bevorzugt in einem Bereich von 45 bis 75 µm und besonders bevorzugt in einem Bereich von 53 bis 63 µm. Gemäß einer bevorzugten Ausführungsform beträgt der Anteil an mikronisiertem Wachs, bezogen auf das Gesamtgewicht an mikronisiertem Wachs und hohlen Glaskügelchen, mehr als 50 Gew.-%, weiterhin bevorzugt mehr als 65 Gew.-%.

Bei einer weiteren Ausführungsform kann der erfindungsgemäßen kosmetischen Formulierung eine Mischung aus gefärbten und ungefärbten mikronisierten Wachsen gleicher oder verschiedener Teilchengröße und/ oder gleicher oder verschiedener chemischer Zusammensetzung der Wachsmatrix zugegeben werden.

Bei einer weiteren Ausführungsform umfasst die erfindungsgemäße kosmetische Formulierung die nichtmetallischen plättchenförmigen synthetischen Substrate ohne weitere Beschichtung und/ oder die nichtmetallischen plättchenförmigen synthetischen Substrate, welche mit einer Oberflächenmodifizierung versehen sind. Sofern es sich bei den nichtmetallischen plättchenförmigen synthetischen Substraten um synthetische Glimmerplättchen handelt, sind diese beispielsweise kommerziell erhältlich als Synafil S 1050 oder Synafil S 525 (beide Fa. Eckart).

**Beispiele**

Die folgenden Beispiele sollen die Erfindung näher erläutern, ohne sie jedoch zu beschränken. Alle Prozentangaben sind als Gew.-% zu verstehen.

**I Beispiele erfindungsgemäßer kosmetischer Formulierungen**

**Beispiel 1** Körper Lotion Wasser-in-Silikon

| INCI Name | Produktname | Gew.- % | Hersteller/ Lieferant |
|---|---|---|---|
| *Phase A* | | | |
| Cyclopentasiloxane (and) Dimethiconol | Dow Corning 1501 | 11,20 | Dow Corning |
| Cyclopentasiloxane | Xiameter PMX-0245 Cyclosiloxane | 5,75 | Dow Corning |
| Cyclopentasiloxane (and) PEG/PPG-18/18 Dimethicone | Dow Corning 5225 C | 13,80 | Dow Corning |
| C 30-45 Alkyl Methicone | Dow Corning Cosmetic Wax AMS-C30 | 3.45 | Dow Corning |
| | **Pigment aus Beispiel 21** | **1,00** | |
| *Phase B* | | | |
| Polysorbate 20 | Tween 20 | 0,60 | Croda |
| Phenoxyethanol (and) Methylparaben (and) Ethylparaben (and) Butylparaben (and) Probylparaben (and) Isobutylparaben | Uniphen P-23 | 0,35 | Induchem |
| Sodium Chloride | Sodium Chloride | 0,75 | VWR |
| Aqua | Wasser | 63,10 | |

Das Pigment aus Beispiel 21 kann in einem Bereich von 0,2 bis 2,5 Gew.-%, bezogen auf das Gesamtgewicht der Körper Lotion Formulierung, eingesetzt werden. Der Ausgleich auf 100 Gew.-% der Formulierung kann mit Wasser erfolgen.

Phase A wurde gemischt und auf 75°C erhitzt, Phase B nach dem Mischen auf 70°C erhitzt, anschließend wurde Phase B langsam unter Homogenisierung zu Phase A hinzugefügt. Unter Rühren wurde die Emulsion abgekühlt und in ein angemessenes Behältnis abgefüllt.

**Beispiel 2** Creme Lidschatten

| INCI Name | Produktname | Gew.-% | Hersteller/ Lieferant |
|---|---|---|---|
| *Phase A* | | | |
| Castor Oil | Castor Oil | 43,70 | Honeywell Riedelde Haen |
| Ethylhexyl Palmitate | Cegesoft C24 | 6,00 | Cognis |
| Cocos Nucifera (Coconut) Oil | Lipovol C-76 | 7,00 | Lipo Chemicals |
| Cera Alba | Ewacera 12 | 6,00 | H. Erhard Wagner |
| Isopropyl Lanolate | Ewalan IP | 5,00 | H. Erhard Wagner |
| Persea Gratissima (Avocado) Oil and Hydrogenated Vegetable Oil | Avocado Butter | 7,00 | Impag |
| Magnesium Stearate | Magnesium Stearate | 3,00 | Sigma-Aldrich |
| Bis-Hydroxyethoxypropyl Dimethicone | Dow Corning 5562 Carbinol Fluid | 7,00 | Dow Corning |
| Dimethicone/ Vinyl Dimethicone Crosspolymer and Silica | Dow Corning 9701 Cosmetic Powder | 5,00 | Dow Corning |
| Phenoxyethanol (and) Methylparaben (and) Ethylparaben (and) Butylparaben (and) Probylparaben (and) Isobutylparaben | Uniphen P-23 | 0,30 | Induchem |
| *Phase B* | | | |
| | **Pigment aus Beispiel 20** | **10,00** | |

Das Pigment aus Beispiel 20 kann in einem Bereich von 5 bis 22,0 Gew.-%, bezogen auf das Gesamtgewicht der Lidschatten Formulierung, eingesetzt werden. Der Ausgleich auf 100 Gew.-% der Formulierung kann mit Castor Oil erfolgen.

Phase A wurde gemischt und auf 85 °C erhitzt, Phase B wurde anschließend unter Rühren zu Phase A hinzugegeben. Nach Abfüllen in ein entsprechendes Behältnis wird die Mischung auf Raumtemperatur abgekühlt.

**Beispiel 3** Duschgel

| INCI Name | Produktname | Gew.-% | Hersteller/ Lieferant |
|---|---|---|---|
| *Phase A* | | | |
| | **Pigment aus Beispiel 21** | **0,50** | |
| Aqua | Wasser | 58,10 | |
| Acrylates Copolymer | Carbopol Aqua SF-1 | 5,50 | Lubrizol |
| *Phase B* | | | |
| Sodium Hydroxide | NaOH (10 Gew.-%) | 1,50 | |
| *Phase C* | | | |
| Sodium Laureth Sulfate | Texapon NSO | 22,00 | Cognis |
| Cocamidopropyl Betaine | Tego Betain F 50 | 6,00 | Evonik |

(fortgesetzt)

| | | | |
|---|---|---|---|
| *Phase C* | | | |
| PEG-7 Glyceryl Cocoate | Emanon HE | 2,00 | Kao Corp. |
| Disodium Laureth Sulfosuccinate | Sectacin 103 Spezial | 2,00 | Zschimmmer & Schwarz |
| *Phase D* | | | |
| Phenoxyethanol (and) Piroctone Olamine | Nipaguard PO 5 | 0,60 | Clariant |
| Fragrance | Water Lily OA | 0,20 | Bell Flavors and Fragrances |
| Sodium Chloride | Sodium Chloride | 1,60 | VWR |

Das Pigment aus Beispiel 21 kann in einem Bereich von 0,01 bis 1,0 Gew.-%, bezogen auf das Gesamtgewicht der Duschgel Formulierung, eingesetzt werden. Der Ausgleich auf 100 Gew.-% der Formulierung kann mit Wasser erfolgen.

Phase A wurde gemischt und gerührt. Danach wurde Phase B zugegeben und gerührt bis ein homogenes Erscheinungsbild erreicht wurde. Phase C wurde separat eingewogen, vermischt und zu Phase AB hinzugefügt. Anschließend wurde erneut gerührt und Phase D einzeln zugegeben.

**Beispiel 4** Gepresster Lidschatten

| INCI Name | Produktname | Gew.-% | Hersteller/ Lieferant |
|---|---|---|---|
| *Phase A* | | | |
| Mica | Silk Mica | 17,00 | VWR |
| Boron Nitride | Softouch CCS 102 | 2,50 | Momentive |
| Zinc Stearate | Zinc Stearate | 7,00 | VWR |
| Talc | Talc Powder | 43,50 | Sigma-Aldrich |
| | **Pigment aus Beispiel 22** | **20,00** | |
| *Phase B* | | | |
| Dimethicone | Xiameter PMX-200 Silicone Fluid 5cs | 5,00 | Dow Corning |
| Cyclopentasiloxane (and) Dimethicone Crosspolymer | Dow Corning 9040 Elastomer | 5,00 | Dow Corning |

Das Pigment aus Beispiel 22 kann in einem Bereich von 5,0 bis 40,0 Gew.-%, bezogen auf das Gesamtgewicht der Lidschatten Formulierung, eingesetzt werden. Der Ausgleich auf 100 Gew.-% der Formulierung kann mit Talc erfolgen.

Phase A wurde für 30s bei 2500 U/min in einem Hochgeschwindigkeitsmixer gemischt. Anschließend wurde Phase B zugegeben und die Mischung für 60s bei 3000 U/min im gleichen Mixer gemischt. Zuletzt wird die Pulvermischung mittels einer Lidschattenpresse bei 150 bar für 30s in Form gepresst.

**Beispiel 5** Haar Mascara

| INCI Name | Produktname | Gew.-% | Hersteller/ Lieferant |
|---|---|---|---|
| *Phase A* | | | |
| Polyquaternium-16 | Luviquat FC 905 (Luviquat Exellence) | 2,70 | BASF |

(fortgesetzt)

| INCI Name | Produktname | Gew.-% | Hersteller/ Lieferant |
|---|---|---|---|
| *Phase A* | | | |
| Propylene Glycol | 1,2-Propanediol | 1,80 | VWR |
| Methylparaben | Methyl-4-hydroxybenzoat e | 0,20 | Sigma-Aldrich |
| Aqua | Wasser | 64,45 | |
| *Phase B* | | | |
| Cetearyl Alcohol | Lanette O | 5,00 | Cognis |
| Dimethicone | Xiameter PMX-200 Silicone Fluid 350cs | 1,00 | Dow Corning |
| Ceteareth-25 | Cremophor A 25 | 2,00 | BASF |
| Propylparaben | Propyl-4-hydroxybenzoat | 0,10 | Sigma-Aldrich |
| *Phase C* | | | |
| Hydroxypropylcellulose | Klucel G | 0,50 | Ashland |
| Magnesium Aluminium Silicate | Veegum HV | 0,50 | R. T. Vanderbilt |
| Aqua | Wasser | 19,00 | |
| *Phase D* | | | |
| | **Pigment aus Beispiel 21** | **2,50** | |
| Phenoxyethanol (and) Methylparaben (and) Butylparaben (and) Ethylparaben (and) Propylparaben (and) Isobutylparaben | Phenonip | 0,20 | Clariant |
| Fragrance | Blue Shadow ÖKO | 0,05 | Bell Flavors and Fragrances |

Das Pigment aus Beispiel 21 kann in einem Bereich von 1,0 bis 10,0 Gew.-%, bezogen auf das Gesamtgewicht der Haar Mascara Formulierung, eingesetzt werden. Der Ausgleich auf 100 Gew.-% der Formulierung kann mit dem Wasser aus Phase A erfolgen.

Phase A und Phase B wurden getrennt auf 80°C erhitzt, danach wurde Phase B langsam zu Phase A hinzugefügt. In einem separaten Gefäß wurden Klucel und Veegum dem Wasser von Phase C hinzugegeben. Anschließend wurde Phase AB auf 40°C abgekühlt und während des Abkühlens Phasen C und D unter Rühren hinzugefügt.

**Beispiel 6** Haargel

| INCI Name | Produktname | Gew.-% | Hersteller/ Lieferant |
|---|---|---|---|
| *Phase A* | | | |
| | **Pigment aus Beispiel 21** | **0,10** | |
| Ammonium Acryloyldimehtyltaurate/ VP Copolymer | Aristoflex AVC | 1,40 | Clariant |
| Citric Acid | Citric Acid | 0,10 | VWR |
| Aqua | Wasser | 55,10 | |

(fortgesetzt)

| Phase B | | | |
|---|---|---|---|
| PVP | Luviskol K 30 Powder | 1,50 | BASF |
| Propylene Glycol, | Germaben II | 0,20 | International |
| Diazolidinyl, Urea, Methylparaben, Propylparaben | | | Speciality Products |
| Triethanolamine | Triethanolamine | 1,20 | VWR |
| Wasser | Aqua | 40,40 | |

Das Pigment aus Beispiel 21 kann in einem Bereich von 0,01 bis 2,0 Gew.-%, bezogen auf das Gesamtgewicht der Haargel Formulierung, eingesetzt werden. Der Ausgleich auf 100 Gew.-% der Formulierung kann mit Wasser erfolgen.

Das Pigment aus Beispiel 21 wurde mit Wasser aus Phase A verrührt, Aristoflex AVC und Citric Acid wurden unter Rühren hinzugegeben und bei einer Geschwindigkeit von 800 U/min für 15 Minuten gemischt. Phase B wurde gelöst bis eine homogene Lösung entstand, anschließend wurde Phase B zu Phase A gegeben und gemischt.

**Beispiel 7** Körperpuder

| INCI Name | Produktname | Gew.-% | Hersteller/ Lieferant |
|---|---|---|---|
| Phase A | | | |
| Mica | Silk Mica | 58,70 | VWR |
| Talc | Talc Powder | 18,00 | Sigma-Aldrich |
| Boron Nitride | Softouch CCS 102 | 5,00 | Advanced Ceramics |
| Nylon-12 | Orgasol 2002 D/Nat | 8,00 | Arkema |
| Magnesium Stearate | Magnesium Stearate | 6,00 | Sigma-Aldrich |
| Methylparaben, Propylparaben | Rokonsal SSH-1 | 0,30 | ISP Biochema |
| | **Pigment aus Beispiel 20** | **2,00** | |
| Phase B | | | |
| Tridecyl Stearate (and) Tridecyl Trimellitate (and) Dipentaerythrityl Hexacaprylate/Hexacaprat e | Lipovol MOS-130 | 2,00 | Lipo Chemicals |

Das Pigment aus Beispiel 20 kann in einem Bereich von 0,2 bis 5,0 Gew.-%, bezogen auf das Gesamtgewicht der Körperpuder Formulierung, eingesetzt werden. Der Ausgleich auf 100 Gew.-% der Formulierung kann mit Silk Mica erfolgen.

Phase A wurde gemischt, anschließend wurde Phase B zu Phase A zugegeben und das Körperpuder in ein geeignetes Gefäß abgefüllt.

**Beispiel 8** Lipgloss

| INCI Name | Produktname | Gew.-% | Hersteller/ Lieferant |
|---|---|---|---|
| Phase A | | | |
| Hydrogenated Polyisobutene (and) Ethylene/ Propylene/Styren e Copolymer (and) Butylene/ Ethylene/Styrene Copolymer | Versagel ME 750 | 79,00 | Calumet Penreco |
| Simmondsia Chinensis (Jojoba) Seed Oil | Jojoba Oil -Natural/Golden | 2,00 | BioChemica |

(fortgesetzt)

| INCI Name | Produktname | Gew.-% | Hersteller/ Lieferant |
|---|---|---|---|
| *Phase A* | | | |
| Caprylyl Trimethicone | Silcare Silicone 31M50 | 7,00 | Clariant |
| Stearyl Dimethicone | Silcare Silicone 41 M65 | 3,20 | Clariant |
| Hydrogenated Polydecene | Nexbase 2002 | 4,00 | Jan Dekker |
| Isopropyl Myristate | Isopropyl Myristate | 4,50 | VWR |
| *Phase B* | | | |
| | **Pigment aus Beispiel 21** | **0,10** | |
| Propylparaben | Propyl-4-hydroxybenzoat | 0,20 | Sigma-Aldrich |

Das Pigment aus Beispiel 21 kann in einem Bereich von 0,10 bis 8,00 Gew.-%, bezogen auf das Gesamtgewicht der Lipgloss Formulierung, eingesetzt werden. Der Ausgleich auf 100 Gew.-% der Formulierung kann mit Versagel ME 750 erfolgen.

Phase A wurde auf 85°C erhitzt, anschließend wurden die Inhaltsstoffe der Phase B einzeln zu Phase A hinzugegeben, gerührt bis eine gleichmäßige Konsistenz entstand und dann in ein Lip Gloss Gefäß abgefüllt.

**Beispiel 9** Lippenkonturenstift

| INCI Name | Produktname | Gew.-% | Hersteller/ Lieferant |
|---|---|---|---|
| *Phase A* | | | |
| Hydrogenated Coco-Glycerides | Softisan 100 | 12,35 | Sasol Wax |
| Candelilla Cera | Ewacera 42 | 14,00 | H. Erhard Wagner |
| Magnesium Stearate | Magnesium Stearate | 6,00 | Sigma-Aldrich |
| Stearic Acid | Kortacid 1895 | 8,50 | Akzo Nobel |
| Hydrogenated Coconut Oil | Lipex 401 | 8,00 | Aarhus Karlshamn |
| Cetyl Palmitate | Kahlwax 7157 | 7,00 | Kahl |
| Caprylic/ Capric Triglyceride | Liponate GC-K | 3,60 | Lipo Chemicals |
| Soybean Glycerides (and) Butyrospermum Parkii | Lipex L'sens | 15,00 | Aarhus Karlshamn |
| Tocopheryl Acetate | dl-alpha-Tocopheryl Acetate | 0,25 | Jan Dekker |
| Methylparaben; Propylparaben | Rokonsal SSH-1 | 0,30 | ISP Biochema |
| *Phase B* | | | |
| | **Pigment aus Beispiel 22** | **25,00** | |

Das Pigment aus Beispiel 22 kann in einem Bereich von 15 bis 25 Gew.-%, bezogen auf das Gesamtgewicht der Lippenkonturenstift Formulierung, eingesetzt werden. Alternativ können zusätzlich zu dem Pigment aus Beispiel 6 weitere Farb-und/ oder Effektpigmente zugegeben werden, die maximale Pigmentierungshöhe von 25 Gew.-% Pigment sollte jedoch nicht überschritten werden.

Phase A wurde auf 85°C erhitzt und anschließend die Phase B der Phase A unter Rühren hinzugefügt bis sich eine gleichförmige Masse ergab. Danach wurde die Mischung heiß in eine Stiftform eingefüllt.

**Beispiel 10** Lippenstift

| INCI Name | Produktname | Gew.-% | Hersteller/ Lieferant |
|---|---|---|---|
| *Phase A* | | | |
| Carnauba Wax | Ewacera 34 | 4,50 | H. Erhard Wagner |
| Cera Alba | Ewacera 12 | 3,50 | H. Erhard Wagner |
| Candelilla Cera Extract | Ewacera 42 | 4,00 | H. Erhard Wagner |
| Microcrystalline Wax | TeCero-Wax 1030 K | 7,20 | TH.C. Tromm |
| Cetyl Palmitate | Kahlwax 7157 | 2,00 | Kahl |
| Hydrogenated Coco-Glycerides | Softisan 100 | 5,00 | Sasol Wax |
| Petrolatum | Penreco Blond | 5,80 | Calumet Penreco |
| Cetearyl Ethylhexanoate | Luvitol EHO | 10,70 | BASF |
| Tocopheryl Acetate | dl-alpha-Tocopheryl Acetate | 0,50 | Jan Dekker |
| Castor Oil | Castor Oil | 46,60 | Honeywell Riedel-de Haen |
| *Phase B* | | | |
| | **Pigment aus Beispiel 20** | **10,00** | |
| Methylparaben, Propylparaben | Rokonsal SSH-1 | 0,20 | ISP Biochema |

Das Pigment aus Beispiel 20 kann in einem Bereich von 0,5 bis 21,0 Gew.-%, bezogen auf das Gesamtgewicht der Lippenstift Formulierung, eingesetzt werden. Der Ausgleich auf 100 Gew.-% der Formulierung kann mit Castor Oil erfolgen.

Phase A wurde auf 85°C erhitzt, danach wurde Phase B zu Phase A gegeben und gemischt. Anschließend wurde diese Mischung bei einer Temperatur von 75°C in einem Lippenstiftform abgefüllt.

**Beispiel 11** Flüssig Lidstrich

| INCI Name | Produktname | Gew.-% | Hersteller/ Lieferant |
|---|---|---|---|
| *Phase A* | | | |
| Aqua | Wasser | 66,70 | |
| Water/ carbon black dispersion | MBD 201 | 3,00 | Geotech |
| Acrylates Copolymer | Covacryl E14 | 10,00 | LCW |
| Magnesium Aluminium Silicate | Veegum HV | 1,00 | C. H. Erbslöh |
| *Phase B* | | | |
| Propylene Glycol | 1,2 Propandiol | 3,00 | VWR |
| Triethanolamine | Triethanolamine | 1,40 | VWR |
| *Phase C* | | | |
| Xanthan Gum | Keltrol CG-T | 0,30 | CP Kelco |

(fortgesetzt)

| | | | |
|---|---|---|---|
| *Phase D* | | | |
| | **Pigment aus Beispiel 22** | **3,00** | |
| Mica | Silk Mica | 2,00 | VWR |
| *Phase E* | | | |
| Stearic Acid | Kortacid 1895 | 2,80 | Akzo Nobel |
| Glyceryl Stearate | Aldo MS K FG | 0,80 | Lonza |
| Oleyl Alcohol | HD-Ocenol 90/95 V | 0,50 | Cognis |
| Phenoxyethanol (and) Methylparaben (and) Ethylparaben (and) Butylparaben (and) Probylparaben (and) Isobutylparaben | Uniphen P-23 | 0,50 | Induchem |
| *Phase F* | | | |
| Dimethicone (and) Trisiloxane | Xiameter PMX-1184 Silicone Fluid | 5,00 | Dow Corning |

Das Pigment aus Beispiel 22 kann in einem Bereich von 0,5 bis 8,0 Gew.-%, bezogen auf das Gesamtgewicht der Lidstrich Formulierung, eingesetzt werden. Der Ausgleich auf 100 Gew.-% der Formulierung kann mit Wasser erfolgen.

Veegum wurde in Phase A dispergiert und 15 Minuten gerührt, danach wurde Phase B zu Phase A hinzugefügt, anschließend Phase C zu Phase AB und erneut 10 Minuten verrührt. Anschließend wurde Phase D zu Phase ABC hinzugefügt und auf 75°C erhitzt, Phase E wurde ebenfalls auf 75°C erwärmt und danach zu Phase ABCD hinzugefügt. Nach dem Abkühlen auf 60°C wurde Phase F zugefügt und in ein geeignetes Gefäß abgefüllt.

**Beispiel 12** Mousse

| INCI Name | Produktname | Gew.-% | Hersteller/ Lieferant |
|---|---|---|---|
| *Phase A* | | | |
| Cyclopentasiloxane | Xiameter PMX-0245 Cyclosiloxane | 8,60 | Dow Corning |
| Hydrogenated Polyisobutene | MC 30 | 4,00 | Sophim |
| Dimethicone (and) Dimethicone Crosspolymer | Dow Corning 9041 Silicone Elastomer Blend | 37,14 | Dow Corning |
| Squalane | Squalane | 5,74 | Impag |
| Isononyl Isononanoate | Dermol 99 | 10,16 | Akzo International |
| Hydrogenated Jojoba Oil | Jojoba Butter LM | 2,15 | Desert Whale |
| Hydrogenated Jojaba Oi | Jojoba Butter HM | 1,00 | Desert Whale |
| C30-45 Alkyl Methicone (and) C30-45 Olefin | Dow Corning AMS-C30 Cosmetic Wax | 1,15 | Dow Corning |
| Stearyl Dimethicone | Dow Corning 2503 Cosmetic Wax | 0,47 | Dow Corning |
| Cyclopentasiloxane (and) Polypropylsilsesqui oxane | Dow Corning 670 Fluid | 5,00 | Dow Corning |

(fortgesetzt)

| Phase B | | | |
|---|---|---|---|
| Dimethicone/ Vinyl Dimethicone Crosspolymer | Dow Corning 9506 Powder | 16,02 | Dow Corning |
| Silica Dimethyl Silylate | Covasilic 15 | 0,17 | LCW |
| Talc | Talc Powder | 5,00 | Sigma-Aldrich |
| | **Pigment aus Beispiel 20** | **3,00** | |
| Phase D | | | |
| Propylene Glycol, Diazolidinyl Urea, Methylparaben, Propylparaben | Germaben II | 0,40 | International Speciality Products |

Das Pigment aus Beispiel 20 kann in einem Bereich von 0,1 bis 8,0 Gew.-%, bezogen auf das Gesamtgewicht der Mousse Formulierung, eingesetzt werden. Der Ausgleich auf 100 Gew.-% der Formulierung kann mit Dow Corning 9041 Elastomer erfolgen.

Phase A wurde gemischt und solange erhitzt bis alles aufgeschmolzen war. Phase B wurden separat eingewogen und mit einem Hochgeschwindigkeitsmixer für 60s bei 2400 U/min gemischt. Die Hälfte der geschmolzenen Phase A wurde zur Phase B zugegeben und wieder im Mixer bei 2400 U/min für 30s gemischt. Anschließend wurde der übrige Teil der Phase B ebenfalls zur Phase A zugegeben und wieder bei 2400 U/min für 30s gemischt. Zuletzt wird Phase C zu Phase AB gegeben und wieder bei 2400 U/min für 30s im Hochgeschwindigkeitsmixer gemischt.

**Beispiel 13** Nagellack

| INCI Name | Produktname | Gew.-% | Hersteller/ Lieferant |
|---|---|---|---|
| Phase A | | | |
| | **Pigment aus Beispiel 20** | **2,00** | |
| Phase B | | | |
| Butylacetat (and) Ethylacetat (and) Nitrocellulose (and) Isopropyl Alcohol | International Lacquers Nailpolish & Care Base 359 | 98,00 | International Lacquers |

Das Pigment aus Beispiel 20 kann in einem Bereich von 0,1 bis 10,0 Gew.-%, bezogen auf das Gesamtgewicht der Nagellack Formulierung, eingesetzt werden. Der Ausgleich auf 100 Gew.-% der Formulierung kann mit International Lacquers Nailpolish erfolgen.

Phase A und Phase B wurden gemischt und anschließend in ein angemessenes Behältnis abgefüllt.

**Beispiel 14** Nagellack mit "soft touch"-Effekt

| INCI Name | Produktname | Gew.-% | Hersteller/ Lieferant |
|---|---|---|---|
| Phase A | | | |
| | **Pigment aus Beispiel 20** | **2,00** | |
| | Ceraflour 913 | 5,00 | Byk Chemie |

(fortgesetzt)

| Phase B | | | |
|---|---|---|---|
| Butylacetat (and) Ethylacetat (and) Nitrocellulose (and) Isopropyl Alcohol | International Lacquers Nailpolish & Care Base 359 | 93,00 | International Lacquers |

Das Pigment aus Beispiel 20 kann in einem Bereich von 0,1 bis 10,0 Gew.-%, bezogen auf das Gesamtgewicht der Nagellack Formulierung, eingesetzt werden. Der Ausgleich auf 100 Gew.-% der Formulierung kann mit International Lacquers Nailpolish erfolgen.

Phase A wurde gemischt, zu Phase B hinzugefügt und anschließend wurde der Nagellack in ein angemessenes Behältnis abgefüllt.

**Beispiel 15** wässriger Nagellack

Die Pigmente aus den Beispielen 20 bis 22 können in einem wässrigen Nagellack gemäß WO 2007/115675 A2 Beispiel 1 eingesetzt werden. Die Pigmentierungshöhe beträgt hierbei 0,1 bis 10,0 Gew.-%, bezogen auf das Gesamtgewicht der Formulierung.

**Beispiel 16** Flüssig Lidschatten

| INCI Name | Produktname | Gew.-% | Hersteller/ Lieferant |
|---|---|---|---|
| Phase A | | | |
| Wasser | Aqua | 70,10 | |
| Glycerin | Pricerine 9090 | 6,00 | Croda |
| Phase B | | | |
| PEG-800 | Polyglycol 35000 S | 0,60 | Clariant |
| Allantoin | Allantoin | 0,30 | 3V |
| Ammonium Acryloyldimehtyltaurate/ VP Copolymer | Aristoflex AVC | 0,80 | Clariant |
| Acrylates Copolymer | Worlee Micromer CEK 20/50 | 5,00 | Worlee |
| Phase C | | | |
| | **Pigment aus Beispiel 22** | **10,00** | |
| Divinyldimethicone/ Dimethicone Copolymer C12-C13 Pareth-3, C12-C13 Pareth-23 | Dow Corning HMW 2220 Non-Ionic Emulsion | 6,00 | Dow Corning |
| Fragrance | Water Lily OA | 0,20 | Bell Flavors and Fragrances |
| Phenoxyethanol (and) Methylparaben (and) Butylparaben (and) Ethylparaben (and) Propylparaben (and) Isobutylparaben | Phenonip | 1,00 | Clariant |

Das Pigment aus Beispiel 22 kann in einem Bereich von 0,10 bis 17,00 Gew.-%, bezogen auf das Gesamtgewicht der Lidschatten Formulierung, eingesetzt werden. Der Ausgleich auf 100 Gew.-% der Formulierung kann mit Wasser erfolgen.

Phase A wurde gerührt, anschließend wurden die Inhaltsstoffe der Phase B einzeln zu Phase A hinzugegeben und gerührt bis eine gleichmäßige Konsistenz entstand. Danach wurden die Inhaltsstoffe der Phase C einzeln zu Phase AB gegeben und gerührt bis wieder eine gleichmäßige Konsistenz entstand.

**II Herstellung der nichtmetallischen plättchenförmigen synthetischen Substrate sowie der silberfarbenen Pigmente für den Einsatz in kosmetischen Formulierungen**

**Beispiel 17**

*Herstellung und Klassierung von synthetischem Glimmer Fluorphlogopit $KMg_3(AlSi_3O_{10})F_2$*

40 Teile wasserfreie Kieselsäure, 30 Teile Magnesiumoxid, 13 Teile Aluminiumoxid und 17 Teile Kaliumhexafluorosilikat wurden gemischt und bei 1500°C geschmolzen. Nach Abkühlen auf 1350°C begann die Kristallisation von Fluorphlogopit ($KMg_3(AlSi_3O_{10})F_2$). Anschließend wurde der Fluorophlogopit zerkleinert und mit einem Laborkoller, Fa. American Cyanamid Company, delaminiert.
Der so erhaltene plättchenförmige Fluorphlogopit wurde in einem Muffelofen bei 1100°C für eine Stunde kalziniert und danach über Laborsiebe klassiert.
Bei der Klassierung wurden zwei Fraktionen mit folgender Partikelgrößenverteilung (MALVERN Mastersizer MS 2000) erhalten:

Fraktion 1: $D_{10}$ = 11,4 $\mu$m, $D_{50}$ = 21,8 $\mu$m, $D_{90}$ = 40,0 $\mu$m,
Fraktion 2: $D_{10}$ = 5,6 $\mu$m, $D_{50}$ = 12,2 $\mu$m, $D_{90}$ = 24,8 $\mu$m

Die Zusammensetzung der synthetischen Glimmerplättchen, gemessen über RFA, ist Tabelle 3 zu entnehmen.

**Beispiel 18**

*Herstellung von synthetischem GlimmerFluorphlogopit $KMg_20,5(AlSi_2O_{10})F_2$*

30 Teile wasserfreie Kieselsäure, 25 Teile Magnesiumoxid, 10 Teile Aluminiumoxid und 15 Teile Kaliumhexafluorosilikat wurden miteinander vermischt und bei 1500°C geschmolzen. Anschließend wurde das flüssige Gemenge bei Temperaturen von 1350°C langsam kristallisiert, um synthetischen Fluorophlogopit ($KMg_20,5(AlSi_2O_{10})F_2$) zu erzeugen. Die erhaltenen synthetischen Glimmerklumpen wurden zerkleinert und im Anschluss daran mit einem Laborkoller, Fa. American Cyanamid Company, delaminiert.
Der so erhaltene plättchenförmige Fluorphlogopit wurde anschließend in einem Muffelofen bei 1100°C für eine Stunde kalziniert und danach über Laborsiebe entsprechend klassiert.
Bei der Klassierung wurden zwei Fraktionen mit folgender Partikelgrößenverteilung (MALVERN Mastersizer MS 2000) erhalten:

Fraktion 1: $D_{10}$ = 10,2 $\mu$m, $D_{50}$ = 20,7 $\mu$m, $D_{90}$ = 42,2 $\mu$m,
Fraktion 2: $D_{10}$ = 6,5 $\mu$m, $D_{50}$ = 13,4 $\mu$m, $D_{90}$ = 25,8 $\mu$m

Die Zusammensetzung der synthetischen Glimmerplättchen, gemessen über RFA, ist Tabelle 3 zu entnehmen.

**Beispiel 19**

*Klassierung von Glasplättchen*

Eine Suspension von 200 g Glasplättchen (mittlere Dicke: 1 $\mu$m, Standardabweichung der Dicke: ca. 40%) in VE-Wasser (ca. 3 Gew.-%ig,VE: vollentsalzt) wurde über ein 100 $\mu$m Sieb klassiert und der Siebdurchgang wiederum über ein 63 $\mu$m Sieb gesiebt. Dieser Siebdurchgang wiederum wurde über ein 36 $\mu$m Sieb gesiebt. Dieser Siebvorgang wurde mit auf dem 36 $\mu$m Sieb erhaltenen Siebrückstand zweimalig wiederholt. So wurde eine Glasplättchenfraktion erhalten, die folgende Partikelgrößenverteilung aufwies (MALVERN Mastersizer MS 2000): $D_{10}$ = 17 $\mu$m, $D_{50}$ = 33 $\mu$m, $D_{90}$ = 59 $\mu$m.

**Beispiel 20**

*Beschichtung des synthetischen Glimmers aus Beispiel 17 mit Ilmenit*

270 g des synthetischen Glimmers aus Beispiel 17 (Fraktion 1) wurden in 1350 ml VE-Wasser suspendiert und unter turbulentem Rühren auf 80°C erhitzt. Der pH-Wert wurde mit verdünnter Salzsäure auf 1,9 gesenkt. Dann wurde eine Schicht "SnO$_2$" auf der Substratoberfläche abgeschieden. Diese Schicht wurde durch Zugabe einer Lösung aus 3 g SnCl$_4$ x 5 H$_2$O (in 10 ml konz. HCl plus 50 ml VE-Wasser) bei gleichzeitiger Zudosierung einer 10%igen wässrigen Natronlauge gebildet. Danach wurde der pH-Wert mit verdünnter Salzsäure auf pH 1,6 heruntergesetzt, sodann eine Lösung aus 400 ml TiCl$_4$ (200 g TiO$_2$/l VE-Wasser) sowie gleichzeitig eine 10%ige wässrige Natronlauge zu der Suspension zudosiert. Nach dem Ende der Beschichtung wurde 1 h nachgerührt und im Anschluss daran der pH-Wert mit verdünnter Natronlauge auf 2,9 eingestellt. Danach wurden 30 ml FeCl$_3$ (280 g Fe$_2$O$_3$/l VE-Wasser) sowie gleichzeitig eine 10%ige wässrige Natronlauge zu der Suspension zudosiert, 1 h nachgerührt, abfiltriert und der Filterkuchen mit VE-Wasser nachgewaschen. Der Filterkuchen wurde bei 800°C unter Formiergasatmosphäre (70% N$_2$/30% H$_2$) 2h in einem Rohrofen kalziniert. Es wurden extrem hochglänzende silberfarbene Pigmente mit metallischem Aussehen erhalten. Die Pigmente wiesen folgende Partikelgrößenverteilung auf (MALVERN Mastersizer MS 2000): D$_{10}$ = 12,4 $\mu$m, D$_{50}$ = 23,9 $\mu$m, D$_{90}$ = 43,1 $\mu$m.

**Beispiel 21**

*Beschichtung des synthetischen Glimmers aus Beispiel 18 mit Ilmenit*

270 g des synthetischen Glimmers aus Beispiel 18 (Fraktion 2) wurden in 2000 ml VE-Wasser suspendiert und unter turbulentem Rühren auf 80°C erhitzt. Der pH-Wert wurde mit verdünnter Salzsäure auf 1,9 gesenkt. Dann wurde eine Schicht "SnO$_2$" auf der Substratoberfläche abgeschieden. Diese Schicht wurde durch Zugabe einer Lösung aus 5 g SnCl$_4$ x 5 H$_2$O (in 10 ml konz. HCl plus 50 ml VE-Wasser) bei gleichzeitiger Zudosierung von einer 10%igen wässrigen Natronlauge gebildet. Danach wurde eine Lösung von 650 ml TiCl$_4$ (200 g TiO$_2$/l VE-Wasser) sowie gleichzeitig eine 10%ige wässrige Natronlauge zu der Suspension hinzudosiert. Nach dem Ende der Beschichtung wurde 1 h nachgerührt und im Anschluss daran der pH-Wert mit verdünnter Natronlauge auf 2,9 eingestellt. Danach wurden 30 ml FeCl$_3$ (280 g Fe$_2$O$_3$/l VE-Wasser) sowie gleichzeitig eine 10%ige wässriger Natronlauge zu der Suspension zudosiert, 1 h nachgerührt, abfiltriert und der Filterkuchen mit VE-Wasser nachgewaschen. Der Filterkuchen wurde bei 800°C unter Formiergasatmosphäre (70% N$_2$/30% H$_2$) 2h in einem Rohrofen kalziniert.
Es wurden glänzende silberfarbene Pigmente mit metallischem Aussehen und hoher Deckfähigkeit erhalten. Die Pigmente wiesen folgende Partikelgrößenverteilung auf (MALVERN Mastersizer MS 2000): D$_{10}$ = 7,3 $\mu$m, D$_{50}$ = 13,3 $\mu$m, D$_{90}$ = 25,4 $\mu$m.

**Beispiel 22**

*Beschichtung der Glasplättchen aus Beispiel 19 mit Ilmenit*

200 g Glasplättchen aus Beispiel 19 wurden in 1800 ml VE-Wasser suspendiert und unter turbulentem Rühren auf 70°C erhitzt. Der pH-Wert wurde mit verdünnter Salzsäure auf 1,9 gesenkt. Dann wurde eine Schicht "SnO$_2$" auf der Substratoberfläche abgeschieden. Diese Schicht wurde durch Zugabe einer Lösung aus 5 g SnCl$_4$ x 5 H$_2$O (in 15 ml konz. HCl plus 65 ml VE-Wasser) unter gleichzeitiger Zudosierung einer 10%igen wässrigen Natronlauge gebildet. Danach wurde 10 min nachgerührt und dann eine Lösung von 100 ml TiCl$_4$ (200 g TiO$_2$/l VE-Wasser) parallel mit 10%iger wässriger Natronlauge in die Suspension hinzudosiert. Nach dem Ende der Beschichtung wurde 1 h nachgerührt und im Anschluss daran der pH-Wert mit verdünnter Natronlauge auf 2,9 eingestellt. Danach wurden 10 ml FeCl$_3$ (280 g Fe$_2$O$_3$/l VE-Wasser) parallel mit 10%iger wässriger Natronlauge in die Suspension hinzudosiert, 1 h nachgerührt, abfiltriert und der Filterkuchen mit VE-Wasser nachgewaschen. Der Filterkuchen wurde bei 550°C unter Formiergasatmosphäre (70% N$_2$/30% H$_2$) 2h in einem Rohrofen kalziniert.
Es wurden extrem hochglänzende, stark glitzernde, silberfarbene Pigmente mit metallischem Aussehen erhalten. Die Pigmente wiesen folgende Partikelgrößenverteilung auf (MALVERN Mastersizer MS 2000): D$_{10}$ = 18,4 $\mu$m, D$_{50}$ = 34,3 $\mu$m, D$_{90}$ = 61,4 $\mu$m.

**Vergleichsbeispiel 1**

*Beschichtung von natürlichem Glimmer Muskovit mit der Partikelgrößenverteilung nach MALVERN Mastersizer MS 2000: D$_{10}$ = 11,0 $\mu$m, D$_{50}$ = 23,1 $\mu$m, D$_{90}$ = 44,4 $\mu$m mit Ilmenit*

Die Beschichtung erfolgte identisch wie in Beispiel 7 aus WO 2004/099319 A2 beschrieben.
Es wurden silberfarbene, deckende Perlglanzpigmente mit geringem Glanz und niedrigem Flopindex erhalten. Die Pigmente wiesen folgende Partikelgrößenverteilung (MALVERN Mastersizer MS 2000): D$_{10}$ = 11,6 $\mu$m, D$_{50}$ = 24,2 $\mu$m, D$_{90}$ = 46,7 $\mu$m.

**Vergleichsbeispiel 2**

Aluminiumeffektpigment Stapa Metallux 2154, Fa. Eckart. Die Pigmente weisen folgende Partikelgrößenverteilung (Cilas 1064) auf: $D_{10}$ = 12,4 $\mu$m, $D_{50}$ = 19,8 $\mu$m, $D_{90}$ = 30,0 $\mu$m.

**Vergleichsbeispiel 3**

Ilmenitbeschichtetes Perlglanzpigment Iriodin 9602 WR, Fa. Merck. Die Pigmente weisen folgende Partikelgrößenverteilung (MALVERN Mastersizer MS 2000): $D_{10}$ = 10,1 $\mu$m, $D_{50}$ = 21,3 $\mu$m, $D_{90}$ = 40,8 $\mu$m.

**Vergleichsbeispiel 4**

Ilmenitbeschichtetes Perlglanzpigment Iriodin 9612 WR, Fa. Merck. Die Pigmente weisen folgende Partikelgrößenverteilung (MALVERN Mastersizer MS 2000): $D_{10}$ = 3,0 $\mu$m, $D_{50}$ = 6,4 $\mu$m, $D_{90}$ = 12,4 $\mu$m.

**Vergleichsbeispiel 5**

Silbernes Perlglanzpigment Phoenix CFE 1001, Fa. Eckart. Die Pigmente weisen folgende Partikelgrößenverteilung (MALVERN Mastersizer MS 2000): $D_{10}$ = 9,6 $\mu$m, $D_{50}$ = 20,3 $\mu$m, $D_{90}$ = 38,3 $\mu$m.

**Vergleichsbeispiel 6**

*Beschichtung von natürlichem Glimmer Muskovit mit der Partikelgrößenverteilung nach MALVERN Mastersizer MS 2000: $D_{10}$ = 11,0 $\mu m$, $D_{50}$ = 23,1 $\mu m$, $D_{90}$ = 44,4 $\mu m$ mit Ilmenit*

Die Beschichtung erfolgte identisch zu Beispiel 1 aus WO 2004/099319 A2.
Es wurden silberfarbene, violettstichige Perlglanzpigmente mit geringem Glanz und niedrigem Flopindex erhalten. Die Pigmente weisen folgende Partikelgrößenverteilung (MALVERN Mastersizer MS 2000): $D_{10}$ = 11,4 $\mu$m, $D_{50}$ = 23,8 $\mu$m, $D_{90}$ = 45,7 $\mu$m.

**Vergleichsbeispiel 7**

Identisch zu Mehrschichtperlglanzpigment aus Beispiel 10 der DE 10 2009 037 935 A1; mittlere Teilchengröße (MALVERN Mastersizer MS 2000): $D_{50}$ = 29,2 $\mu$m.

**Vergleichsbeispiel 8**

Identisch zu Perlglanzpigment aus Beispiel 1a der DE 10 2009 049 413 A1; mittlere Teilchengröße (MALVERN Mastersizer MS 2000): $D_{50}$ = 3,2 $\mu$m.

**III Charakterisierung der nichtmetallischen plättchenförmigen synthetischen Substrate und der silberfarbenen Pigmente**

Die Charakterisierung erfolgte wie nachfolgend beschrieben anhand des silberfarbenen Pigments.

**IIIa Teilchengrößenmessung**

Die Größenverteilungskurve der nichtmetallischen plättchenförmigen synthetischen Substrate sowie der Pigmente wurde mit einem Gerät der Fa. Malvern (Gerät: MALVERN Mastersizer 2000) gemäß Herstellerangaben bestimmt. Hierzu wurden ca. 0,1 g des entsprechenden Substrates bzw. Pigmentes als wässrige Suspension, ohne Zusatz von Dispergierhilfsmitteln, unter ständigem Rühren mittels einer Pasteurpipette in die Probenvorbereitungszelle des Messgerätes gegeben und mehrmals vermessen. Aus den einzelnen Messergebnissen wurden die resultierenden Mittelwerte gebildet. Die Auswertung der Streulichtsignale erfolgte dabei nach der Fraunhofer Methode.
Die Größenverteilungskurve des Metalleffektpigments (in Pastenform) aus Vergleichsbeispiel 2, der wurde mit einem Gerät der Fa. Quantachrome (Gerät: Cilas 1064) gemäß Herstellerangaben vermessen. Hierzu wurden ca. 1,5 g des Pigmentes in Isopropanol suspendiert, 300 Sekunden im Ultraschallbad (Gerät: Sonorex IK 52, Fa. Bandelin) behandelt und anschließend mittels einer Pasteurpipette in die Probenvorbereitungszelle des Messgerätes gegeben und mehrmals vermessen. Aus den einzelnen Messergebnissen wurden die resultierenden Mittelwerte gebildet. Die Auswertung der Streulichtsignale erfolgte dabei nach der Fraunhofer Methode.

Unter der mittleren Größe $D_{50}$ wird im Rahmen dieser Erfindung der $D_{50}$-Wert der Summenhäufigkeitsverteilung der volumengemittelten Größenverteilungsfunktion, wie sie durch Laserbeugungsmethoden erhalten werden, verstanden. Der $D_{50}$-Wert gibt an, dass 50 % der nichtmetallischen plättchenförmigen synthetischen Substrate bzw. Pigmente einen Durchmesser aufweisen, der gleich oder kleiner als der angegebene Wert, beispielsweise 20 $\mu$m, ist. Entsprechend gibt der $D_{90}$-Wert an, dass 90% der Substrate bzw. Pigmente einen Durchmesser aufweisen, der gleich oder kleiner als der jeweilige Wert ist. Weiterhin gibt der $D_{10}$-Wert an, dass 10% der Substrate bzw. Pigmente einen Durchmesser aufweisen, der gleich oder kleiner als der jeweilige Werte ist.

**IIIb Bestimmung der mittleren Dicke der nichtmetallischen plättchenförmigen synthetischen Substrate**

Zur Bestimmung der mittleren Dicke der nichtmetallischen plättchenförmigen synthetischen Substrate wurden die Substrate oder die Pigmente 10 Gew.-%ig in einem 2K Klarlack Autoclear Plus HS, Fa. Sikkens, mit einem Hülsenpinsel eingearbeitet und mit Hilfe einer Spiralrakel (26 $\mu$m Nassfilmdicke) auf eine Folie appliziert und getrocknet. Nach 24h Trocknung wurden von diesen Rakelabzügen Querschliffe angefertigt, die rasterelektronenmikroskopisch vermessen wurden. Hierbei wurden mindestens 100 Pigmentteilchen vermessen, um eine aussagefähige Statistik zu erhalten. Die mittlere Dicke der als Substrat eingesetzten synthetischen Glimmerplättchen ist Tabelle 2 zu entnehmen.

Tabelle 2

|  | Beispiel 17 Fraktion 1 Mittlere Dicke [nm] | Beispiel 17 Fraktion 2 Mittlere Dicke [nm] | Beispiel 18 Fraktion 1 Mittlere Dicke [nm] | Beispiel 18 Fraktion 2 Mittlere Dicke [nm] |
|---|---|---|---|---|
| $D_{10}$ | 289 | 154 | 321 | 138 |
| $D_{50}$ | 434 | 219 | 443 | 209 |
| $D_{90}$ | 734 | 318 | 779 | 294 |

Die Angabe $D_{10}$ bedeutet hier, dass 10% der nichtmetallischen plättchenförmigen synthetischen Substrate eine mittlere Dicke aufweisen, welche gleich oder kleiner als der angegebene Wert ist. Entsprechend gibt hier der $D_{50}$ bzw. $D_{90}$-Wert an, dass 50% bzw. 90% der nichtmetallischen plättchenförmige synthetischen Substrate eine mittlere Dicke aufweisen, welche gleich oder kleiner als der angegebene Wert ist.

**IIIc Bestimmung des Metalloxidgehaltes**

Die Metalloxidgehalte der nichtmetallischen plättchenförmigen synthetischen Substrate bzw. der Pigmente wurden mittels Röntgenfluoreszenzanalyse (RFA) bestimmt.
Hierzu wurde das Substrat bzw. Pigment in eine Lithiumtetraboratglastablette eingearbeitet, in Festprobenmessbechern fixiert und daraus vermessen. Als Messgerät diente das Gerät Advantix ARL, Fa. Thermo Scientific

Tabelle 3: Metalloxidgehalte gemäß RFA der als Substrat eingesetzten synthetischen Glimmerplättchen

| Metalloxid | Beispiel 1 (Gew.-%) | Beispiel 2 (Gew.%) |
|---|---|---|
| $TiO_2$ | < 0,1 | < 0,1 |
| $SnO_2$ | < 0,1 | < 0,1 |
| $SiO_2$ | 42,1 | 20,3 |
| $Al_2O_3$ | 12,2 | 8,1 |
| $K_2O$ | 11,0 | 15,9 |
| $Fe_2O_3$ | 0,1 | 0,1 |
| $Cr_2O_3$ | < 0,1 | < 0,1 |
| $CeO_2$ | < 0,1 | < 0,1 |
| $CaO$ | 0,1 | < 0,1 |
| $MgO$ | 31,6 | 27,6 |
| $Na_2O$ | 0,2 | 0,4 |

(fortgesetzt)

| Metalloxid | Beispiel 1 (Gew.-%) | Beispiel 2 (Gew.%) |
|---|---|---|
| $P_2O_5$ | < 0,1 | < 0,1 |
| MnO | < 0,1 | < 0,1 |

Die in Tabelle 3 angegebenen Gew.-%-Angaben beziehen sich jeweils auf das Gesamtgewicht des nichtmetallischen plättchenförmigen Substrats.

Tabelle 4: Magnesiumoxidgehalt der Pigmente gemäß RFA

| | MgO (Gew.-%) |
|---|---|
| Beispiel 20 | 19,6 |
| Beispiel 21 | 17,1 |
| Vergleichsbeispiel 1 | 0,3 |
| Vergleichsbeispiel 3 | 0,3 |
| Vergleichsbeispiel 4 | 0,2 |
| Vergleichsbeispiel 5 | 0,3 |

Die in Tabelle 4 angegebenen Gew.-%-Angaben beziehen sich jeweils auf das Gesamtgewicht des jeweiligen Pigments.

EP 2 502 966 A1

Tabelle 5: Eisen/ Titan-Gewichtsverhältnis der Pigmente

| | Beispiel 20 | Beispiel 21 | Beispiel 22 | Vergleichsbeispiel 1 | Vergleichsbeispiel 3 | Vergleichsbeispiel 4 | Vergleichsbeispiel 6 |
|---|---|---|---|---|---|---|---|
| $Fe_2O_3$ (Gew.-%) | 2,9 | 3,2 | 2,8 | 4,3 | 5,9 | 9,9 | 3,7 |
| $FeTiO_3$ (Gew.-%) | 5,4 | 6,1 | 5,3 | 8,1 | 11,3 | 18,8 | 7,1 |
| Fe (Gew.-%), berechnet | 2,0 | 2,2 | 2,0 | 3,0 | 4,1 | 6,9 | 2,6 |
| $TiO_2$ (Gew.-%) | 23,9 | 33,4 | 9,7 | 30,2 | 24,7 | 30,4 | 28,0 |
| Fe/ Ti-Gewichts-verhältnis | 0,14 | 0,11 | 0,34 | 0,17 | 0,28 | 0,38 | 0,16 |
| Fe/ Ti-Gewichts-verhältnis (Schicht) | 4,37 | 4,21 | 4,60 | 6,16 | 9,54 | 16,28 | 5,01 |

Die in Tabelle 5 angegebenen Gewichtsanteile beziehen sich jeweils auf das Gesamtgewicht des Pigmentes.

Bei den in Tabelle 5 angegebenen Eisenwerten handelt es sich um auf elementares Eisen umgerechnete Werte. Hierzu wurden die Gehalte aller mittels RFA im Pigment nachweisbaren Eisenverbindungen auf elementares Eisen umgerechnet. Zur Berechnung des in Tabelle 5 angegebenen Fe/ Ti-Gewichtsverhältnisses wurde der mittels RFA gemessene Titanoxidgehalt des Pigments auf elementares Titan umgerechnet.

Bei dem in Tabelle 5 angegeben Fe/ Ti-Gewichtsverhältnis (Schicht) wurde der Anteil der Beschichtung der Pigmente gemäß

$$\frac{\text{Eisengehalt (Gew. -\%)}}{\text{Titangehalt (Gew. -\%)}} \cdot \text{Anteil der Beschichtung (Gew.-\%)}$$

berücksichtigt. Der Anteil der Beschichtung (Gew.-%) definiert sich aus dem Gesamtgewicht des Pigments (100 Gew.-%) abzüglich des Anteils des Substrates (Gew.-%).

**IIId Bestimmung der Bleigehalte über Feststoff AAS**

Die Bleigehalte der synthetischen Glimmerplättchen bzw. der auf synthetischen Glimmerplättchen basierenden Pigmente wurden über die Feststoff-Graphitrohr-Atomabsorptionsspektrometrie bestimmt. Als Gerät wurde ein ZEENIT 650 mit Feststoffprobengeber SSA 600 (Hersteller: Analytik Jena) eingesetzt. Die entsprechenden Gehalte der synthetischen Glimmerplättchen bzw. der darauf basierenden Pigmente sind Tabelle 6 zu entnehmen.

Tabelle 6

|  | Bleigehalt [ppm] |
|---|---|
| Beispiel 17 | < 1 |
| Beispiel 18 | < 1 |
| Beispiel 20 | < 1 |
| Beispiel 21 | < 1 |

**IIIe Bestimmung der Chemikalienbeständigkeit**

Die Chemikalienbeständigkeit der Pigmente aus den Beispielen und Vergleichsbeispielen wurde anhand von Lackapplikationen auf Blechen bestimmt. 6 g des jeweiligen Pigments (in Pulverform) wurden in eine Mischung aus 90 g eines konventionellen Nasslacks auf Basis hydroxyfunktioneller Acrylate (CSR-Lack, farblos) und 10 g Butylacetat 85 eingerührt. Danach wurde die Viskosität mit einem Gemisch aus Butylacetat 85 und Xylol im Verhältnis 1:1 auf 17" im DIN 4 mm-Becher eingestellt.

Jeweils 100 g dieses Lackes wurden analog zu IVa mit einem Spritzautomaten auf die Bleche deckend appliziert. Nach der Beschichtung wurden die Bleche 30 min bei 80°C eingebrannt.

24 h später wurden auf das Blech je ein Tropfen 10 Gew.-%ige HCl sowie ein Tropfen einer 1 M Natronlauge versetzt aufgebracht. Nach einer Einwirkzeit von jeweils 0,5h, 1 h, 2h und 3h wurden die HCl- bzw. NaOH-Tropfen mit VE-Wasser abgewaschen und die Bleche je nach Beschädigung der Lackschicht visuell beurteilt. Dabei wurde eine extrem starke Schädigung, d.h. eine vollständige Auflösung des Pigmentes mit 10 und kein Unterschied zum unbehandelten Blech mit 0 bewertet. Die Ergebnisse dieser visuellen Beurteilung sind in Tabelle 7 wiedergegeben.

Tabelle 7: Chemikalienbeständigkeit

| Pigment gemäß | Chemikalienbeständigkeit | | |
|---|---|---|---|
|  | Säure | Lauge | Summe |
| Beispiel 20 | 0 | 0 | 0 |
| Beispiel 21 | 0 | 2 | 2 |
| Beispiel 22 | 0 | 1 | 1 |
| Vergleichsbeispiel 1 | 0 | 4 | 4 |

(fortgesetzt)

| Pigment gemäß | Chemikalienbeständigkeit | | |
|---|---|---|---|
| | Säure | Lauge | Summe |
| Vergleichsbeispiel 2 | 5 | 10 | 15 |
| Vergleichsbeispiel 3 | 0 | 6 | 6 |
| Vergleichsbeispiel 4 | 3 | 4 | 7 |
| Vergleichsbeispiel 5 | 0 | 0 | 0 |
| Vergleichsbeispiel 6 | 0 | 3 | 3 |

Die silberfarbenen Pigmente sowie das herkömmliche transparente silberfarbene Perlglanzpigment der Phoenixserie der Fa. Eckart (Vergleichsbeispiel 5) zeichnen sich durch ihre extrem hohe Chemikalienbeständigkeit aus.

**IIIf Temperaturbeständigkeit**

Zur Überprüfung der Temperaturbeständigkeit wurden die Pigmente jeweils 30 min bei Temperaturen von 100°C und 200°C gelagert. Anhand von Rakelabzügen des jeweiligen Pigments in einem konvenionellen Nitrocelluloselack (Dr. Renger Erco Bronzemischlack 2615e; Fa. Morton, Pigmentierungshöhe von 10 Gew.-%, bezogen auf das Gesamtgewichtes des Nasslacks) auf Schwarz-Weiß-Deckungskarten (Byko-Chart 2853, Fa. Byk Gardner) wurden eventuell auftretende Farbveränderungen visuell begutachtet.
Hierbei wurde festgestellt, dass bei Rakelabzügen der silberfarbenen Pigmenten weder nach Lagerung der Pigmente bei 100°C noch nach Lagerung der Pigmente bei 200°C eine Farbänderung zu beobachten war.

**IIIg Helligkeit L\***

Die Helligkeit L* der nichtmetallischen plättchenförmigen synthetischen Substrate wurde durch diffuse Farbmessung der jeweiligen Pulverschüttungen mit dem Farbmessgerät CR 310, Firma Konica Minolta, gemessen.

Tabelle 8

| | Helligkeit L* diffus |
|---|---|
| Beispiel 1 | 97,6 |
| Beispiel 2 | 98,4 |
| Beispiel 3 | 96,7 |

**IIIh Diffuse Farbmessung**

Die Helligkeit L*, a*- und b*-Werte und das Chroma wurden durch diffuse Farbmessung der jeweiligen Pulverschüttungen mit einem Farbmessgerät CM700d der Firma Konica Minolta bestimmt.

Tabelle 9

| Beispiel / Vergleichsbeispiel | L* | a* | b* | C* | h° |
|---|---|---|---|---|---|
| Beispiel 22 | 48,9 | -0,3 | 0,1 | 0,3 | 165,2 |
| Vergleichsbeispiel 7 | 49,5 | 12,4 | 14,3 | 19,0 | 49,2 |
| Vergleichsbeipiel 3 | 60,8 | -0,5 | 1,5 | 1,6 | 108,1 |
| Vergleichsbeispiel 4 | 50,6 | -1,6 | -4,1 | 4,4 | 249,4 |

Das erfindungsgemäße Beispiel 22 zeichnet sich durch niedrige a*- und b*-Werte und folglich auch durch niedrige Chromawerte aus. Das Mehrschichtperlglanzpigment aus Vergleichsbeispiel 7 weist zwar eine silberne Interferenzfarbe auf, dennoch erkennt man auch an dem Chromawert deutlich, dass das Pigment eine rötlich braune Absorptionsfarbe aufweist. Auch bei den Vergleichsbeispielen 3 und 4 ist im Unterschied zu dem erfindungsgemäßen Beispiel 22 immer wenigstens einer der Werte C*, a* oder b* erhöht, so dass auch hier messtechnisch der visuell wahrnehmbare Farbstich belegt werden konnte.

**IIIi Korrosionsbeständigkeit**

Die Korrosionsbeständigkeit der silberfarbenen Pigmente der kosmetischen Formulierung wurde durch Bestimmung des Gasungsverhaltens in einem wässrigen Carbomergel-System bestimmt. Hierzu wurde zunächst ein Carbomergel bestehend aus 0,7 Gew.-% Gelbildner Aristoflex AVC, Fa. Clariant, und 99,3 Gew.-% VE-Wasser unter Rühren hergestellt. Anschließend wurde eine Suspension aus 23 Gew.-% silberfarbenen Pigments und 77 Gew.-% VE-Wasser unter Rühren zu dem Carbomergel gegeben. Dieser Mischung wurde 1 Gew.-%, bezogen auf deren Gesamtgewicht, des Konservierungsmittel Uniphen P-23 hinzugefügt, um eine Verkeimung und gegebenenfalls eine Verfälschung des Messergebnisses durch Gasausscheidung der Mikroben zu unterbinden. Im Anschluß wurden 300g der so erhaltenen Mischung in eine Gaswaschflasche gefüllt, mit einem Doppelkammergasblasenzähler verschlossen und anschließend im Wasserbad auf 40°C erwärmt. Die Gasentwicklung wurde über einen Zeitraum von 30 Tagen ermittelt. Nach diesen 30 Tagen war bei den silberfarbenen Pigmenten keine Gasentwicklung zu beobachten. Der Test gilt als bestanden, wenn nach 30 Tagen eine Gasentwicklung von < 10mL auftritt. Im Idealfall ist keine Gasentwicklung zu beobachten.

**IV Charakterisierung des optischen Effekts der silberfarbenen Pigmente**

Die Charakterisierung erfolgte wie nachfolgend beschrieben anhand des silberfarbenen Pigments.

**IVa Bestimmung des Hell-/ Dunkelflops (Flopindex)**

Der Flopindex der Pigmente aus den Beispielen und Vergleichsbeispielen wurde anhand von Lackapplikationen auf Blechen bestimmt. 6 g des jeweiligen Pigments (in Pulverform) wurden in eine Mischung aus 90 g eines konventionellen Nasslack auf Basis hydroxyfunktioneller Acrylate (CSR-Lack, farblos) und 10 g Butylacetat 85 eingerührt. Danach wurde die Viskosität mit 25 g eines Gemisch aus Butylacetat 85 und Xylol im Verhältnis 1:1 auf 17" im DIN 4 mm-Becher eingestellt. Jeweils 100g dieses Lackes wurden mit einem Spritzautomaten und der Spritzpistole LP-90, Einstellung Nadel 1.3.5 (beides Fa. Languth) bei einem Druck von 4 bar auf Bleche appliziert (6 Gänge). Nach 15 min Ablüftzeit wurde eine weitere Klarlackschicht (70g KL Autoclear Plus und 42g Härter P25, jeweils Fa. Sikkens) bei einem Druck von 4 bar in 3 Gängen aufgebracht (Einstellung Nadel: 2.0.3). Die Bleche wurden nach der Beschichtung 30 min bei 80°C eingebrannt.

Der Flopindex ist nach Alman folgendermaßen definiert (S. Schellenberger, M. Entenmann, A. Hennemann, P. Thometzek, Farbe und Lack, 04/2007, S. 130):

$$\text{Flopindex} = 2{,}69 \cdot (L_{E1} - L_{E3})^{1,11} / L_{E2}^{0,86}$$

wobei $L_{E1}$ für die Helligkeit des glanznahen Messwinkels (E1 = 15° zum Glanzwinkel), $L_{E2}$ für die Helligkeit des Messwinkels zwischen glanznahem und glanzfernen Winkel (E2 = 45° zum Glanzwinkel) und $L_{E3}$ für die Helligkeit des glanzfernen Messwinkels (E3 = 110° zum Glanzwinkel) steht.

Je größer der Zahlenwert des Flopindexes ist, desto stärker kommt der Hell-/Dunkelflop zum Ausdruck.

Zur Bestimmung des Flopindex wurde die Helligkeit L* mittels Mehrwinkelfarbmessung mit dem Gerät Bykmac, Fa. Byk Gardner, vermessen. Die entsprechenden Werte sind in Tabelle 10 aufgeführt.

Beispiel 20 und 22 weisen sogar einen höheren Flopindex als ein vergleichbares Metallpigment (Vergleichsbeispiel 2) auf. Beispiel 21 besitzt einen niedrigeren Flopindex, was auf die kleinere Teilchengröße und die damit einhergehende stärkere Streuung zurückzuführen ist. Verglichen zu einem Metalleffektpigment mit ähnlicher Korngrößenverteilung ist auch hier der Flopindex des silberfarbenen Pigmentes leicht über dem des vergleichbaren Metalleffektpigmentes.

Das Vergleichsbeispiel 3 besitzt einen vergleichbar hohen Flopindex. Dieser wird durch die stärkere Färbung des Pigmentes begünstigt. Optisch ist das Pigment jedoch nicht farbneutral (Chroma) und somit als Imitat bzw. Ersatz eines Alumiumeffektpigments nahezu ungeeignet.

**IVb Effektmessungen**

Effektmessungen zur Bestimmung des Glitzereffekts der Pigmente wurden anhand der Spritzapplikationen aus IVa mit einem BYK-mac (Fa. Byk-Gardner) durchgeführt.

Zur Simulation von Effektänderungen bei direkter Beleuchtung wird der Glitzereffekt mit dem BYK-mac unter Verwendung einer hochauflösenden CCD-Kamera untersucht. Der Glitzereffekt, verursacht durch das Reflexionsvermögen der einzelnen Effektpigmente, wird nur bei direkter Sonneneinstrahlung wahrgenommen und verändert sich in Abhängigkeit des Beleuchtungswinkels. Aus diesem Grund wird die Probe bei dem Byk-mac mit sehr hellen LEDs unter drei verschiedenen Winkeln (15°/45°/75°) beleuchtet. Mit der CCD-Kamera wird dabei senkrecht zur Oberfläche jeweils ein Bild aufgenommen. Die Bilder werden mit Hilfe von Bildverarbeitungsalgorithmen analysiert, wobei das Histogramm der Helligkeitsstufen als Basis für die Berechnung der Glitzerparameter verwendet wird. Um eine bessere Differenzierung zu gewährleisten, wurde der Glitzereffekt durch ein zweidimensionales System beschrieben, der Glitzerfläche S_a und der Glitzerintensität S_i. Alternativ wurden genannte Angaben durch den eindimensionalen Wert, den Glitzergrad S_G zusammengefasst. Die entsprechenden Messwerte sind in Tabelle 10 zusammengefasst.

Entscheidend für den optischen Eindruck ist der eindimensionale Glitzergrad S_G. Je höher der Zahlenwert von S_G, desto höher ist der auch vom Auge wahrnehmbare Glitzereffekt. In einer zweidimensionalen Darstellung kann der Glitzergrad S_G in die Komponenten Glitzerintensität S_i und Glitzerfläche S_a aufgeteilt werden. Da beide Komponenten einen maßgeblichen Einfluss auf den Glitzergrad S_G haben, kann es vorkommen, dass ein Effektpigment in den Messgeometrien 15°, 45° und 75° nahezu den gleichen Glitzergrad S_G aufweist, obwohl die Zahlenwerte von S_a und S_G in den betrachteten Messgeometrien deutlich erhöht bzw. erniedrigt sind.

Die silberfarbenen Pigmente sind hinsichtlich ihrer Glitzerintensität S_i Perlglanzpigmenten, welche auf natürlichen Glimmerplättchen basieren, überlegen. Bei einem Vergleich von Glitzerfläche S_a, Glitzerintensität S_i, Glitzergrad S_G und Flopindex ist die mittlere Teilchengröße $D_{50}$ zu berücksichtigen. Es sind also nur Pigmente gleicher oder ähnlicher mittlerer Teilchengröße untereinander vergleichbar. Eine geringere mittlere Teilchengröße $D_{50}$, wie bei Beispiel 21, äußert sich in geringeren Werten für Glitzerfläche S_a, Glitzerintensität S_i, Glitzergrad S_G und Flopindex.

Die in Tabelle 10 dargestellten Messwerte für Helligkeit, Chroma, Flopindex, S_g, S_a, S_i wurden anhand der Spritzapplikationen auf IIIa bestimmt.

Tabelle 10: Helligkeit L*, Chroma, Flopindex, Glitzerfläche S_a, Glitzerintensität S_i, Glitzergrad S_G der Pigmente

| | Messgeometrie | Helligkeit L* | Chroma | Flopindex | S_G [15°] | S_i [15°] | S_a [15°] |
|---|---|---|---|---|---|---|---|
| Beispiel 20 | 15° | 132,8 | 0,5 | 21,4 | 6,7 | 14,7 | 31,1 |
| | 25° | 92,0 | 0,8 | | | | |
| | 45° | 45,3 | 1,3 | | | | |
| | 75° | 26,1 | 1,9 | | | | |
| | 110° | 20,2 | 1,6 | | | | |
| Beispiel 21 | 15° | 120,3 | 0,67 | 15,8 | 4,4 | 9,1 | 24,1 |
| | 25° | 92,7 | 0,51 | | | | |
| | 45° | 53,4 | 0,91 | | | | |
| | 75° | 30,8 | 1,21 | | | | |
| | 110° | 22,9 | 0,93 | | | | |
| Beispiel 22 | 15° | 116,0 | 0,4 | 20,0 | 12,3 | 33,0 | 21,2 |
| | 25° | 78,6 | 0,9 | | | | |
| | 45° | 37,5 | 1,7 | | | | |
| | 75° | 27,1 | 2,1 | | | | |
| | 110° | 24,4 | 1,6 | | | | |
| Vergleichsbeispiel 1 | 15° | 126,0 | 1,0 | 16,8 | 3,7 | 7,7 | 24,1 |
| | 25° | 95,8 | 0,5 | | | | |
| | 45° | 53,3 | 0,8 | | | | |
| | 75° | 30,7 | 1,0 | | | | |
| | 110° | 23,2 | 0,8 | | | | |

(fortgesetzt)

| | Messgeometrie | Helligkeit L* | Chroma | Flopindex | S_G [15°] | S_i [15°] | S_a [15°] |
|---|---|---|---|---|---|---|---|
| Vergleichsbeispiel 2 | 15° | 149,8 | 0,4 | 19,1 | 5,2 | 11,0 | 26,6 |
| | 25° | 109,7 | 0,36 | | | | |
| | 45° | 56,9 | 0,50 | | | | |
| | 75° | 34,0 | 0,73 | | | | |
| | 110° | 28,5 | 1,22 | | | | |
| Vergleichsbeispiel 3 | 15° | 120,3 | 1,76 | 20,2 | 4,7 | 10,0 | 24,7 |
| | 25° | 87,0 | 2,10 | | | | |
| | 45° | 43,4 | 2,36 | | | | |
| | 75° | 23,3 | 2,68 | | | | |
| | 110° | 16,9 | 2,41 | | | | |
| Vergleichsbeispiel 4 | 15° | 83,8 | 1,41 | 10,3 | 0,8 | 2,7 | 7,9 |
| | 25° | 72,5 | 0,82 | | | | |
| | 45° | 50,4 | 1,69 | | | | |
| | 75° | 30,7 | 2,57 | | | | |
| | 110° | 20,3 | 2,15 | | | | |
| Vergleichsbeispiel 5 | 15° | 140,4 | 1,5 | 9,8 | 3,8 | 8,1 | 20,9 |
| | 25° | 109,6 | 1,3 | | | | |
| | 45° | 72,3 | 2,1 | | | | |
| | 75° | 59,5 | 3,1 | | | | |
| | 110° | 60,1 | 2,5 | | | | |
| Vergleichsbeispiel 6 | 15° | 125,8 | 0,5 | 16,2 | 3,1 | 6,6 | 18,3 |
| | 25° | 96,5 | 0,9 | | | | |
| | 45° | 54,2 | 1,8 | | | | |
| | 75° | 31,8 | 2,6 | | | | |
| | 110° | 24,9 | 2,1 | | | | |
| Vergleichsbeispiel 7 | 15° | 97,1 | 18,0 | 11,6 | 6,6 | 13,7 | 32,5 |
| | 25° | 69,8 | 28,5 | | | | |
| | 45° | 43,3 | 49,6 | | | | |
| | 75° | 36,7 | 59,5 | | | | |
| | 110° | 34,5 | 62,1 | | | | |
| Vergleichsbeispiel 8 | 15° | 119,8 | 1,2 | 1,9 | 0,9 | 2,1 | 7,8 |
| | 25° | 105,8 | 0,9 | | | | |
| | 45° | 84,7 | 1,2 | | | | |
| | 75° | 73,7 | 0,2 | | | | |
| | 110° | 72,0 | 0,7 | | | | |

Tabelle 11

|  | Flopindex/D50 | Flopintensität $F_I$ = (Flopindex. S_i)/D50 |
|---|---|---|
| Beispiel 20 | 0,9 | 13,2 |
| Beispiel 21 | 1,2 | 10,8 |
| Beispiel 22 | 0,6 | 19,2 |
| Vergleichsbeispiel 1 | 0,7 | 5,3 |
| Vergleichsbeispiel 2 | 1,0 | 10,6 |
| Vergleichsbeispiel 3 | 1,0 | 9,5 |
| Vergleichsbeispiel 4 | 1,6 | 4,3 |
| Vergleichsbeispiel 5 | 0,5 | 3,9 |
| Vergleichsbeispiel 6 | 0,7 | 4,5 |
| Vergleichsbeispiel 7 | 0,4 | 5,4 |
| Vergleichsbeispiel 8 | 0,6 | 1,3 |

Die Werte der Flopintensität aller Vergleichsbeispiele mit Ausnahme des Vergleichsbeispiels 2 liegen <10 und weisen somit optisch keinen ausreichenden Metallcharakter auf. Bei Vergleichsbeispiel 2 handelt es sich um ein Metalleffektpigment. Die in der erfindungsgemäßen kosmetischen Formulierung einzusetzenden silberfarbenen Pigmente aus den Beispielen 20 bis 22 besitzen Flopintensitäten von >10 und zeigen deshalb hervorragenden metallischen Charakter.

**IVc Glanzmessungen**

Der Glanz ist ein Maß für die gerichtete Reflexion und lässt sich mittels eines Micro-Tri-Gloss-Gerätes genau charakterisieren. Stärker streuende Proben sollten aufgrund von vermehrter Kantenstreuung sowie Pigmentunebenheiten einen niedrigen Glanz aufweisen.
Es wurden die Lackapplikationen auf Schwarz-Weiß-Deckungskarten mit Hilfe eines Micro-Tri-Gloss Glanzmessgerätes, Fa. Byk Gardner, bei einem Messwinkel von 60° bezogen auf die Vertikale vermessen. Die jeweiligen Pigmente wurden in einen konventionellen Nitrocelluloselack (Dr. Renger Erco Bronzemischlack 2615e; Fa. Morton, Pigmentierungshöhe 10 Gew.-%, bezogen auf das Gesamtgewicht des Nitrocelluloselacks) eingerührt. Der fertige Lack wurde mit einem Rakelabzugsgerät mit einer Nassfilmdicke von 76 μm auf Schwarz-Weiß-Deckungskarten (Byko-Chart 2853, Fa. Byk-Gardner) appliziert.
Die nachstehend in Tabelle 12 aufgeführten Glanzwerte stellen Mittelwerte aus jeweils fünf Einzelmessungen dar.
Die silberfarbenen Pigmente weisen einen deutlich stärkeren Glanz als die Vergleichsbeispiele auf. Eine Ausnahme stellt das Vergleichsbeispiel 5 dar. Aufgrund der hohen Transparenz dieses Pigmentes geht der weiße Untergrund der Deckungskarte in die Messung mit ein.

**IVd Deckfähigkeit**

Die Deckfähigkeit der Pigmente aus den Beispielen und Vergleichsbeispielen wurde anhand der Lackapplikationen auf Schwarz-Weiß-Deckungskarten aus IVc bestimmt. Die Helligkeitswerte L* wurden bei einer Messgeometrie von 110°, relativ zum Ausfallwinkel des bei 45° eingestrahlten Lichts, anhand dieser Lackapplikationen auf dem schwarzem und auf dem weißem Hintergrund der Schwarz-Weiß-Deckungskarte mit dem Gerät BYK-mac, Fa. Byk Gardner, vermessen.
Durch Bildung des Deckungsquotienten Dq lassen sich vom Untergrund unabhängige Maßzahlen für die Deckfähigkeit der Pigmente ermitteln. Hierzu wird der Quotient der Helligkeitswerte auf schwarzem Hintergrund zu den Helligkeitswerten auf weißem Hintergrund der Schwarz-Weiß-Deckungskarte berechnet:

$$Dq = \frac{L^*_{110,schwarz}}{L^*_{110,weiß}}$$

Bei Verwendung identischer Lacksysteme erlaubt der Deckungsquotient den Vergleich der Deckfähigkeit von verschiedenen Effektpigmenten zueinander.

Die silberfarbenen Perlglanzpigmente aus Beispiel 20 erreichen eine vergleichbare Deckfähigkeit zu Aluminiumeffektpigmenten gleicher mittlerer Teilchengröße (Vergleichsbeispiel 2).

Die Vergleichsbeispiele 3 und 4 besitzen zwar eine sehr gute Deckfähigkeit, wirken aufgrund des sehr geringen Glanzes sowie der hohen Chromawerte jedoch keinesfalls metallisch und sind optisch extrem unattraktiv.

Tabelle 12: Deckungsquotient und Glanz

|  | Deckungsquotient 110° Byk-mac | Glanz [60°] |
|---|---|---|
| Beispiel 4 | 0,630 | 32,1 |
| Beispiel 5 | 0,760 | 19,6 |
| Beispiel 6 | 0,527 | 23,0 |
| Vergleichsbeispiel 1 | 0,524 | 25,3 |
| Vergleichsbeispiel 2 | 1,001 | 19,8 |
| Vergleichsbeispiel 3 | 0,654 | 17,5 |
| Vergleichsbeispiel 4 | 0,938 | 17,4 |
| Vergleichsbeispiel 5 | 0,393 | 38,5 |

**Patentansprüche**

1. Kosmetische Formulierung umfassend ein kosmetisch akzeptables Medium und wenigstens ein silberfarbenes Pigment, wobei das silberfarbene Pigment ein nichtmetallisches plättchenförmiges Substrat und wenigstens eine ilmenithaltige Beschichtung umfasst.

2. Kosmetische Formulierung gemäß Anspruch 1,
**dadurch gekennzeichnet,**
**dass** das nichtmetallische plättchenförmige Substrat des silberfarbenen Pigments ein nichtmetallisches plättchenförmiges synthetisches Substrat ist und der Gehalt an Eisenverbindungen, berechnet als elementares Eisen, in dem Pigment bei weniger als 5,0 Gew.-%, bezogen auf das Gesamtgewicht des silberfarbenen Pigmentes, liegt.

3. Kosmetische Formulierung gemäß einem der vorstehenden Ansprüche,
**dadurch gekennzeichnet,**
**dass** das silberfarbene Pigment einen Eisen(III)oxid-Gehalt von weniger als 0,5 Gew.-%, bezogen auf das Gesamtgewicht des Pigmentes, enthält.

4. Kosmetische Formulierung gemäß einem der vorstehenden Ansprüche,
**dadurch gekennzeichnet,**
**dass** das silberfarbene Pigment eine Flopintensität $F_i$ definiert als Produkt aus Flopindex und Glitzerintensität S_i in Abhängigkeit von der mittleren Teilchengröße $D_{50}$ gemäß Formel (II)

$$\text{Flopintensität}(F_i) = \frac{Flopindex \cdot S\_i}{D_{50}} \qquad (II)$$

von wenigstens 10 aufweist.

5. Kosmetische Formulierung gemäß einem der vorstehenden Ansprüche,
**dadurch gekennzeichnet,**
**dass** das Chroma des silberfarbenen Pigmentes bei einer Messgeometrie von 110°, relativ zum Ausfallwinkel des bei 45° eingestrahlten Lichts, bei $C^*_{110} \leq 2,4$ liegt.

6. Kosmetische Formulierung gemäß einem der vorstehenden Ansprüche,
**dadurch gekennzeichnet,**
**dass** die Ilmenitschicht des silberfarbenen Pigments eine mittlere Schichtdicke aus einem Bereich von 1 nm bis 20 nm aufweist.

7. Kosmetische Formulierung gemäß einem der vorstehenden Ansprüche,
**dadurch gekennzeichnet,**
**dass** das silberfarbene Pigment ein Eisen/ Titan-Gewichtsverhältnis in Abhängigkeit von der Beschichtung gemäß Formel (I)

$$\frac{\text{Eisengehalt (Gew.-\%)}}{\text{Titangehalt (Gew.-\%)}} \cdot \text{Anteil der Beschichtung (Gew.-\%)} \qquad \text{(I)}$$

in einem Bereich von 1 bis 8 aufweist,
wobei "Eisengehalt" für den Gehalt an Eisenverbindungen, berechnet als elementares Eisen, und "Titangehalt" für den Gehalt an Titanverbindungen, berechnet als elementares Titan, jeweils im Pigment und bezogen auf das Gesamtgewicht des silberfarbenen Pigmentes steht, und wobei der "Anteil der Beschichtung (Gew.-%)" für den auf das Gesamtgewicht des Pigmentes bezogenen Gewichtsanteil der auf das Substrat aufgebrachten Beschichtung steht.

8. Kosmetische Formulierung gemäß einem der vorstehenden Ansprüche,
**dadurch gekennzeichnet,**
**dass** das silberfarbene Pigment folgenden Aufbau umfasst:

(a) nichtmetallisches plättchenförmiges synthetisches Substrat,
(b) Titanoxidschicht,
(c) Ilmenitschicht,

wobei das Pigment erhältlich ist durch

(i) Aufbringen einer nicht kalzinierten Titanoxid-/ Titanhydroxid-/Titanoxidhydratschicht auf einem nichtmetallischen, plättchenförmigen, synthetischen Substrat,
(ii) Aufbringen einer Eisenoxid-/ Eisenhydroxid-/ Eisenoxidhydratschicht auf die nicht kalzinierte Titanoxid-/ Titanhydroxid-/ Titanoxidhydratschicht,
(iii) Kalzinieren des in Schritt (ii) erhaltenen Produktes unter reduzierenden Bedingungen.

9. Kosmetische Formulierung gemäß einem der vorstehenden Ansprüche,
**dadurch gekennzeichnet,**
**dass** das nichtmetallische plättchenförmige synthetische Substrat des silberfarbenen Pigments aus der Gruppe, bestehend aus synthetischen Glimmerplättchen, Glasplättchen, $SiO_2$-Plättchen, $Al_2O_3$-Plättchen, synthetischen Böhmitplättchen, Polymerplättchen, synthetischen plättchenförmigen Substraten, die eine anorganisch-organische Mischschicht umfassen, und deren Gemische, ausgewählt ist.

10. Verwendung eines silberfarbenen Pigments umfassend ein nichtmetallisches plättchenförmiges Substrat und wenigstens eine ilmenithaltige Beschichtung in einer kosmetischen Formulierung.

11. Verwendung eines silberfarbenen Pigments nach Anspruch 10,
**dadurch gekennzeichnet,**
**dass** das nichtmetallische plättchenförmige Substrat des silberfarbenen Pigments ein nichtmetallisches plättchenförmiges synthetisches Substrat ist und der Gehalt an Eisenverbindungen, berechnet als elementares Eisen, in dem Pigment bei weniger als 5,0 Gew.-%, bezogen auf das Gesamtgewicht des silberfarbenen Pigmentes, liegt.

12. Verwendung eines silberfarbenen Pigments nach einem der Ansprüche Anspruch 10 oder 11,
**dadurch gekennzeichnet,**
**dass** die Ilmenitschicht des silberfarbenen Pigments eine mittlere Schichtdicke aus einem Bereich von 1 nm bis 20 nm aufweist.

**13.** Verwendung eines silberfarbenen Pigments nach einem der Ansprüche Anspruch 10 bis 12,
**dadurch gekennzeichnet,**
**dass** das silberfarbene Pigment ein Eisen/ Titan-Gewichtsverhältnis in Abhängigkeit von der Beschichtung gemäß Formel (I)

$$\frac{\text{Eisengehalt (Gew.-\%)}}{\text{Titangehalt (Gew.-\%)}} \cdot \text{Anteil der Beschichtung (Gew.-\%)} \qquad (I)$$

in einem Bereich von 1 bis 8 aufweist,
wobei "Eisengehalt" für den Gehalt an Eisenverbindungen, berechnet als elementares Eisen, und "Titangehalt" für den Gehalt an Titanverbindungen, berechnet als elementares Titan, jeweils im Pigment und bezogen auf das Gesamtgewicht des silberfarbenen Pigmentes steht, und wobei der "Anteil der Beschichtung (Gew.-%)" für den auf das Gesamtgewicht des Pigmentes bezogenen Gewichtsanteil der auf das Substrat aufgebrachten Beschichtung steht.

**14.** Verwendung eines silberfarbenen Pigments nach einem der Ansprüche Anspruch 10 bis 13,
**dadurch gekennzeichnet,**
**dass** das silberfarbene Pigment folgenden Aufbau umfasst:

(a) nichtmetallisches plättchenförmiges synthetisches Substrat,
(b) Titanoxidschicht,
(c) Ilmenitschicht,

wobei das Pigment erhältlich ist durch

(i) Aufbringen einer nicht kalzinierten Titanoxid-/ Titanhydroxid-/Titanoxidhydratschicht auf einem nichtmetallischen, plättchenförmigen, synthetischen Substrat,
(ii) Aufbringen einer Eisenoxid-/ Eisenhydroxid-/ Eisenoxidhydratschicht auf die nicht kalzinierte Titanoxid-/ Titanhydroxid-/ Titanoxidhydratschicht,
(iii) Kalzinieren des in Schritt (ii) erhaltenen Produktes unter reduzierenden Bedingungen.

**15.** Verwendung eines silberfarbenen Pigments nach einem der Ansprüche Anspruch 10 bis 14,
**dadurch gekennzeichnet,**
**dass** das silberfarbene Pigment eine Flopintensität $F_i$ definiert als Produkt aus Flopindex und Glitzerintensität S_i in Abhängigkeit von der mittleren Teilchengröße $D_{50}$ gemäß Formel (II)

$$\text{Flopintensität}(F_i) = \frac{Flopindex \cdot S\_i}{D_{50}} \qquad (II)$$

von wenigstens 10 aufweist.

Abbildung 1:

Abbildung 2: Byk mac Effekt-Meßgeometrien (Byk-Gardner, Katalog
"Qualitätskontrolle für Lacke und Kunststoffe" 2011/2012, S. 97)

Europäisches
Patentamt

European
Patent Office

Office européen
des brevets

## EUROPÄISCHER RECHERCHENBERICHT

Nummer der Anmeldung

EP 12 16 1116

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 2004/099319 A2 (MERCK PATENT GMBH [DE]; KNIESS HELGE BETTINA [DE]; PFAFF GERHARD [DE]) 18. November 2004 (2004-11-18) * Seite 5, Zeile 19 - Zeile 23 * * Seite 8, Zeile 20 - Zeile 32 * * Seite 9, Zeile 11 - Zeile 16 * * Seite 17, Zeile 20 - Seite 18, Zeile 2; Anspruch 4; Beispiele 1,7 * ----- | 1-15 | INV. C09C1/00 A61K8/02 A61K8/19 A61Q1/02 |
| X | DE 103 31 903 A1 (MERCK PATENT GMBH [DE]) 26. Februar 2004 (2004-02-26) * Absätze [0009], [0011], [0012], [0015], [0043] * ----- | 1,8-10, 14 | |

RECHERCHIERTE
SACHGEBIETE (IPC)

C09C
A61K
A61Q

Der vorliegende Recherchenbericht wurde für alle Patentansprüche erstellt

| Recherchenort | Abschlußdatum der Recherche | Prüfer |
|---|---|---|
| München | 14. Juni 2012 | King, Ruth |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument

   ...............................................................
& : Mitglied der gleichen Patentfamilie, übereinstimmendes
   Dokument

EPO FORM 1503 03.82 (P04C03)

EP 2 502 966 A1

## ANHANG ZUM EUROPÄISCHEN RECHERCHENBERICHT
### ÜBER DIE EUROPÄISCHE PATENTANMELDUNG NR.

EP 12 16 1116

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-06-2012

| Im Recherchenbericht angeführtes Patentdokument | Datum der Veröffentlichung | Mitglied(er) der Patentfamilie | Datum der Veröffentlichung |
|---|---|---|---|
| WO 2004099319 A2 | 18-11-2004 | CN 1784476 A<br>DE 10320455 A1<br>EP 1620511 A2<br>JP 2006526041 A<br>US 2007028799 A1<br>WO 2004099319 A2 | 07-06-2006<br>25-11-2004<br>01-02-2006<br>16-11-2006<br>08-02-2007<br>18-11-2004 |
| DE 10331903 A1 | 26-02-2004 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0289240 A1
- WO 2004056716 A1
- WO 2005063637 A1
- EP 1980594 B1
- DE 102011001579
- WO 2004099319 A2

- EP 0246523 A2
- US 20110052804 A1
- WO 2007115675 A2
- DE 102009037935 A1
- DE 102009049413 A1

**In der Beschreibung aufgeführte Nicht-Patentliteratur**

- *FDA Code of Federal Regulation Title 21,* vol. 1 (73), 1645
- **NANCY M. HEPP ; WILLIAM R. MINDAK ; JOHN CHENG.** *J. Cosmet. Sci.,* Juli 2009, vol. 60, 405-414

- **BYK-GARDNER.** *Qualitätskontrolle für Lacke und Kunststoffe,* 2011, 97, 98